# EUROPEAN PATENT SPECIFICATION

(11) **EP 2 180 028 B1**
(45) Date of publication and mention of the grant of the patent: **16.10.2013**
(21) Application number: 08827334.7
(22) Date of filing: 07.08.2008
(51) Int. Cl.: C09K 3/18, A61K 8/19, A61K 8/25, A61Q 1/02, A61Q 1/12, C09C 1/00, C09C 3/12, C09C 1/28

(54) **SURFACE TREATING METHOD, SURFACE-TREATED POWDER AND COSMETIC PREPARATION**
OBERFLÄCHENBEHANDLUNGSMETHODE, OBERFLÄCHENBEHANDELTES PULVER UND KOSMETISCHE ZUBEREITUNG
PROCÉDÉ DE TRAITEMENT DE SURFACE, POUDRE TRAITÉE EN SURFACE ET PRÉPARATION COSMÉTIQUE

(30) Priority: 10.08.2007 JP 2007210331
(43) Date of publication of application: 28.04.2010
(73) Proprietor: Shiseido Company, Ltd., Chuo-ku Tokyo 104-8010 (JP); Dow Corning Toray Co., Ltd., Tokyo, 100-0004 (JP)
(72) Inventor: IKEDA, Tomoko, Yokohama-shi Kanagawa 224-8558 (JP); NAKAMA, Yasunari, Yokohama-shi Kanagawa 224-8558 (JP); KANEKO, Katsuyuki, Yokohama-shi Kanagawa 224-8558 (JP); NISHIHAMA, Shuji, Yokohama-shi Kanagawa 224-8558 (JP); IIMURA, Tomohiro, Ichihara-shi Chiba 299-0108 (JP); OKAWA, Tadashi, Ichihara-shi Chiba 299-0108 (JP)
(74) Representative: Lippert, Stachow & Partner
(86) International application number: PCT/JP2008/064200
(87) International publication number: WO 2009/022621

(56) References cited:
- JP-A- 5 034 312
- JP-A- 8 109 263
- JP-A- 05 320 234
- JP-A- 63 199 273
- JP-A- 2004 026 669
- JP-A- 2006 213 885
- JP-B2- 3 450 541

## Description

### FIELD OF THE INVENTION

The present invention relates to a surface treating method, a surface-treated powder, and a cosmetic, particularly to improvement of the water resistance, sebum resistance, and texture in use of a surface-treated powder incorporated in a cosmetic.

### BACKGROUND OF THE INVENTION

Heretofore, organo(poly)siloxanes having various organic groups introduced in a portion of their structures have been developed by use of properties specific to dimethylpolysiloxane typified by silicone oil. Such organo(poly)siloxanes have low surface tension and a low refractive index and further have properties in combination, such as low susceptibility to friction, heat resistance, cold resistance, antistatic properties, water repellency, mold releasability, anti-foaming properties, and chemical resistance. Therefore, they are used in various fields. There exist organo(poly)siloxanes modified at various functional groups or at structural positions for introduction thereof, according to the usage.

Various compounds have been developed and studied so far, for example, as organo(poly)siloxane derivatives containing a carboxyl group, which is a hydrophilic organic group. As typical examples, organosiloxane derivatives having a carboxyl group introduced in the side chain of a linear polysiloxane structure are widely known. In recent years, siloxane dendrimers containing a carboxyl structure have also been reported as one example of such compounds (see e.g., Patent Literatures 1 to 3). Furthermore, it has also been reported that a compound obtained by neutralizing carboxyl-modified silicone with triethanolamine has an emulsification capacity (see e.g., Non-Patent Literatures 1 and 2).

On the other hand, makeup cosmetics containing a powder, for example, foundations and eye shadows are required to have various performances such as spreadability over the skin, adhesion to the skin, and long-lasting finish. Thus, an attempt has been made widely to prevent makeup from coming off due to sweat, sebum, or the like and improve long-lasting finish effects by treating powders for cosmetics with surface treating agents. Organo(poly)siloxanes have heretofore been used as these surface treating agents for powders for cosmetics. For example, a technique has been reported, which comprises: treating a powder with organosiloxane having a carboxyl group introduced in the side chain or either end of dimethylpolysiloxane as a basic skeleton; and formulating, in a cosmetic, the powder thus having water repellency (see e.g., Patent Literature 4). However, even powders surface-treated with the conventional organo(poly)siloxane are not sufficiently satisfactory in terms of all of water resistance, sebum resistance, texture in use, and so on. Thus, further improvement thereof has been demanded.

Patent Literature 1: Japanese Unexamined Patent Publication 2000-072784
Patent Literature 2: Japanese Unexamined Patent Publication 2000-239390
Patent Literature 3: Japanese Unexamined Patent Publication 2001-213885
Patent Literature 4: Japanese Patent No. 3450541
Non-patent Literature 1: Kazuki KAGESHIMA and Toshiyuki SHIMIZU, "Application of carboxyl-modified silicone as surfactant in emulsification," Fragrance Journal extra edition 19 (2005): 125 - 130
Non-patent Literature 2: Kazuki KAGESHIMA, Harumi SAKAMOTO, and Toshiyuki SHIMIZU, "Application of carboxyl-modified silicone as surfactant in cosmetic field," Journal of SCCJ Vo1.34 No.4 (2003): 309 - 314
JP 8 109263 A discloses an organosiloxane having a divalent or trivalent metal salt of either one or two terminal carboxyl groups being obtained by reacting the respective sodium carboxylate salt with a higher valent salt solution.
JP 05034312 discloses a polyorganosiloxane which contains a carboxylate group -COO⁻ nMⁿ⁺, connected to a silicon atom.

### DISCLOSURE OF THE INVENTION

### PROBLEM TO BE SOLVED BY THE INVENTION

The present invention has been conducted in view of the problems of the prior art, and an object of the present invention is to provide a surface treating method to obtain a surface treated powder which can improve texture in use and impart excellent water resistance and sebum resistance.

### MEANS TO SOLVE THE PROBLEM

To solve the problems of the prior art, the present inventors have studied diligently and consequently completed the present invention by finding that a surface-treated powder that has improved texture in use and is very excellent in water resistance and sebum resistance is obtained by using, as a surface treating agent, a specifically structured organosiloxane derivative containing a carboxyl group and surface-treating a powder with the organopolysiloxane derivative.

Specifically, a surface treating method according to the present invention is characterized by using a composition comprising an organosiloxane derivative having a divalent or trivalent metal salt of a terminal carboxyl group, represented by the following formula (1) or (3).

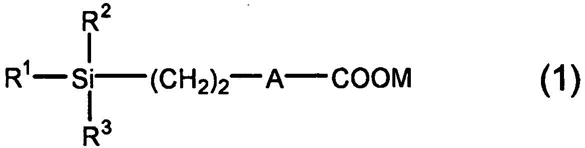

In the formula (1), at least one of R¹ to R³ is a functional group represented by -O-Si(R⁴)₃ in which R⁴ is an alkyl group having 1 to 6 carbon atoms or a phenyl group, or a functional group represented by -O-Si(R⁵)₂-X¹ in which R⁵ is an alkyl group having 1 to 6 carbon atoms or a phenyl group, and X¹ is a functional group represented by the following formula (2) when i=1; and the remaining R¹ to R³ may be the same or different and each may be a substituted or unsubstituted monovalent hydrocarbon group; M is a divalent or trivalent metal atom; A is a linear or branched alkylene group represented by C_{q}H_{2q} in which q is any integer of 0 to 20; and the organosiloxane derivative represented by the formula (1) contains a total of 2 to 100 silicon atoms (Si) on average per molecule.

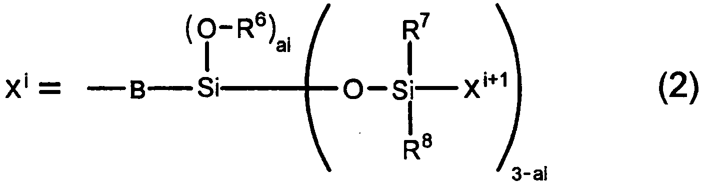

In the formula (2), R⁶ is a hydrogen atom, an alkyl group having 1 to 6 carbon atoms, or a phenyl group; R⁷ and R⁸ are respectively an alkyl group having 1 to 6 carbon atoms or a phenyl group; B is a linear or branched alkylene group represented by CᵣH₂ᵣ which may be partially branched in which r is any integer of 2 to 20; and i specifies the generation of a silylalkyl group represented by Xⁱ and is any integer of 1 to n when the generation number is n, wherein the generation number n is any integer of 1 to 10; ai is any integer of 0 to 2 when i is 1, and is an integer smaller than 3 when i is 2 or larger; and Xⁱ⁺¹ is the silylalkyl group when i is smaller than n, and is a methyl group when i=n.

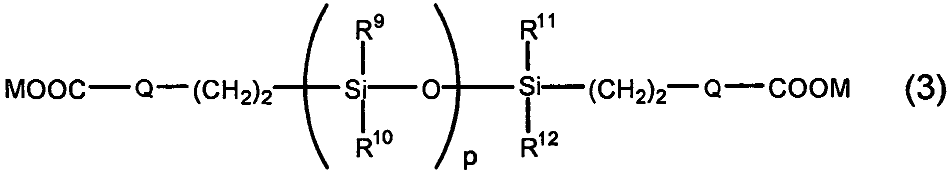

In the formula (3), R⁹ to R¹² may be the same or different and are respectively a substituted or unsubstituted monovalent hydrocarbon group; M is a divalent or trivalent metal atom; Q is a linear or branched alkylene group represented by C_{q}H_{2q} in which q is any integer of 0 to 20; and p is any number of 0 to 20.

Moreover, a surface treating agent as used according to the present invention is characterized by being obtained by mixing an organosiloxane derivative having a terminal carboxyl group or a monovalent metal salt thereof, represented by the following formula (1') or (3'), with a metal salt solution containing a divalent or trivalent metal ion.

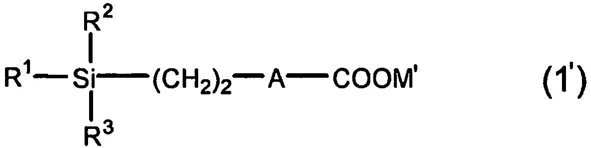

In the formula (1'), R¹ to R³ and A are as defined above; M' is a hydrogen atom or a monovalent metal atom; and the organosiloxane derivative represented by the formula (1') contains a total of 2 to 100 silicon atoms (Si) on average per molecule.

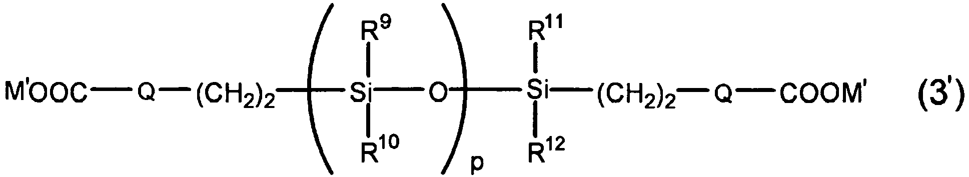

In the formula (3'), R⁹ to R¹² and Q are as defined above; P is as defined above; and M' is a hydrogen atom or a monovalent metal atom.

Moreover, in the surface treating agent, it is preferred that the organosiloxane derivative should be represented by the formula (1) or (1'), wherein R¹ and R² are respectively a functional group represented by -O-Si(R⁴)₃ in which R⁴ is an alkyl group having 1 to 6 carbon atoms; R³ is a monovalent hydrocarbon group having 1 to 10 carbon atoms; and q is any integer of 6 to 20.
Moreover, in the surface treating agent, it is preferred that the organosiloxane derivative should be represented by the formula (1) or (1'), wherein at least one or more of R¹ to R³ are respectively any of the functional group represented by the following formula (4) or (5), and the remaining R¹ to R³ may be the same or different and are respectively a substituted or unsubstituted monovalent hydrocarbon group.

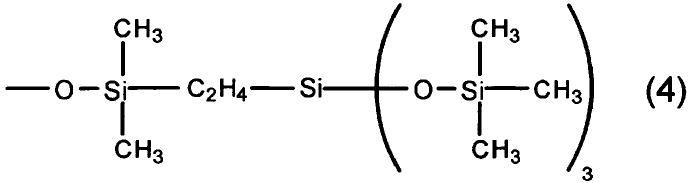

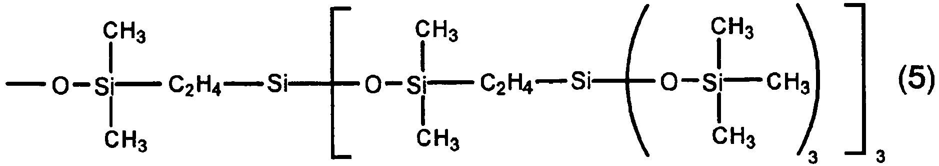

Moreover, in the surface treating agent, it is preferred that the organosiloxane derivative should be represented by the formula (3) or (3'), wherein R⁹ to R¹² are respectively a group selected from the group consisting of substituted or unsubstituted alkyl groups having 1 to 20 carbon atoms, aryl groups, and aralkyl groups; q is any integer of 6 to 20; and p is any number of 1 to 20.

Moreover, a surface-treated powder according to the present invention is characterized by being treated with the surface treating agent.
Moreover, a cosmetic according to the present invention is characterized by comprising the surface-treated powder.

### EFFECT OF THE INVENTION

According to the present invention, a surface-treated powder that has improved texture in use and is very excellent in water resistance and sebum resistance is obtained by using, as a surface treating agent, a specifically structured organosiloxane derivative containing a carboxyl group and surface-treating a powder with the organopolysiloxane derivative.

### BEST MODE FOR CARRYING OUT THE INVENTION

Hereinafter, the preferred embodiments of the present invention will be described.
A surface treating agent as used according to the present invention is characterized by comprising an organosiloxane derivative having a divalent or trivalent metal salt of a terminal carboxyl group, or by being obtained by mixing an organosiloxane derivative having a terminal carboxyl group or a monovalent metal salt thereof, with a metal salt solution containing a divalent or trivalent metal ion.

### Organosiloxane derivative

The organosiloxane derivative used in the present invention is a compound represented by the formula (1) or (1'), or the above-mentioned formula (3) or (3').
First, the organosiloxane derivative represented by the following formula (1) will be described.

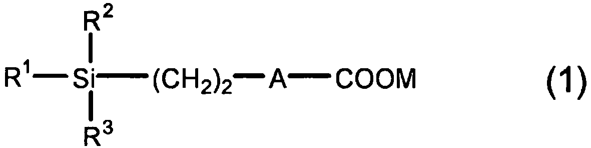

The organosiloxane derivative represented by the formula (1) is an organosiloxane derivative modified with an alkylcarboxyl group and is characterized by containing a total of 2 to 100 silicon atoms on average per molecule.
In the formula (1), at least one of R¹ to R³ is a functional group represented by -O-Si(R⁴)₃ in which R⁴ is an alkyl group having 1 to 6 carbon atoms or a phenyl group, or a functional group represented by -O-Si(R⁵)₂-X¹ in which R⁵ is an alkyl group having 1 to 6 carbon atoms or a phenyl group, and X¹ is a functional group represented by the following formula (2) when i=1. In this context, all of R¹ to R³ may respectively be any of the functional groups. Alternatively, when at least one of R¹ to R³ is any of the functional groups, the remaining R¹ to R³ may be the same or different and each may be a substituted or unsubstituted monovalent hydrocarbon group.

In the functional group represented by -O-Si(R⁴)₃, R⁴ is an alkyl group having 1 to 6 carbon atoms or a phenyl group. Examples of the alkyl group having 1 to 6 carbon atoms include linear, branched, or cyclic alkyl groups such as methyl, ethyl, n-propyl, i-propyl, n-butyl, i-butyl, s-butyl, t-butyl, pentyl, neopentyl, cyclopentyl, and hexyl. Examples of the functional group represented by -O-Si(R⁴)₃ include -O-Si(CH₃)₃, -O-Si(CH₃)₂(C₂H₅), -O-Si(CH₃)₂(C₃H₇), -O-Si(CH₃)₂(C₄H₉), -O-Si(CH₃)₂(C₅H₁₁), -O-Si(CH₃)₂(C₆H₁₃), -O-Si(CH₃)₂(C₆H₅). In this context, the functional group is preferably a trialkylsiloxy group, most preferably a trimethylsiloxy group.

Moreover, the functional group represented by -O-Si(R⁵)₂-X¹ is an organosiloxy group having a dendrimer structure. R⁵ is an alkyl group having 1 to 6 carbon atoms or a phenyl group. Moreover, X¹ is a functional group represented by the following formula (2) when i=1.

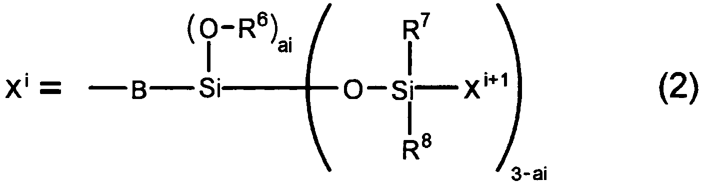

In the formula (2), R⁶ is a hydrogen atom, an alkyl group having 1 to 6 carbon atoms, or a phenyl group, and R⁷ and R⁸ are respectively an alkyl group having 1 to 6 carbon atoms or a phenyl group. R⁶ to R⁸ are respectively preferably an alkyl group having 1 to 6 carbon atoms, particularly preferably a methyl group. Moreover, B is a linear or branched alkylene group represented by CᵣH₂ᵣ which may be partially branched, and r is any integer of 2 to 20. Examples of the alkylene group having 2 to 20 carbon atoms, represented by B include: linear alkylene groups such as ethylene, propylene, butylene, and hexylene groups; and branched alkylene groups such as methylmethylene, methylethylene, 1-methylpentylene, and 1,4-dimethylbutylene groups. Among them, an ethylene or hexylene group is preferable.

In the formula (2), i specifies the generation of a silylalkyl group represented by Xⁱ and is any integer in the range of 1 to n when the generation number, i.e., the number of repetitions of the silylalkyl group, is n. The generation number n is any integer of 1 to 10. Xⁱ⁺¹ is the silylalkyl group when i is smaller than n, and is a methyl group (-CH₃) when i=n. ai is any integer of 0 to 2 when i=1, and is a number smaller than 3 when i is 2 or larger. ai is preferably 1 or smaller, particularly preferably 0.
Specifically, when the generation n of the dendrimer structure is 1, the silylalkyl group of the formula (2) is represented by the following formula.

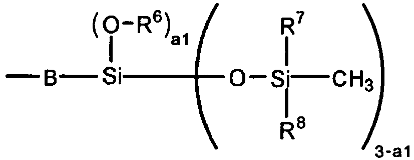

When the generation n of the dendrimer structure is 2, the silylalkyl group of the formula (2) is represented by the following formula.

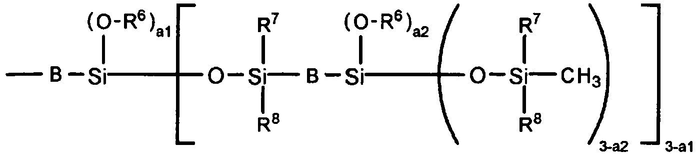

When the generation n of the dendrimer structure is 3, the silylalkyl group of the formula (2) is represented by the following formula.

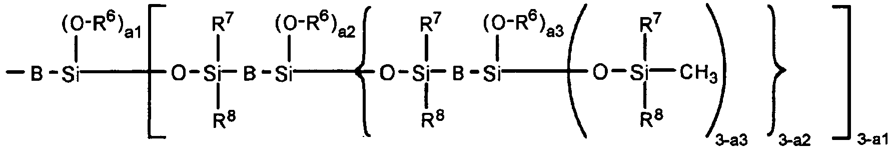

Particularly preferable examples of the functional group represented by -O-Si(R⁵)₂-X¹ include a functional group represented by the following formula (4) wherein the generation number n of the silylalkyl group is 1, a functional group represented by the following formula (5) wherein the generation number n of the silylalkyl group is 2, and a functional group represented by the following formula (6) wherein the generation number n of the silylalkyl group is 3.

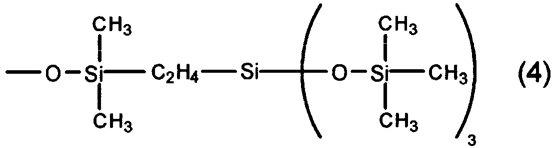

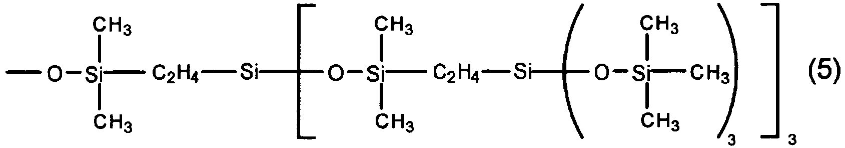

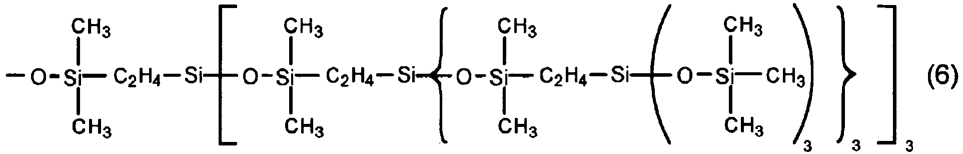

Moreover, in the formula (1), as long as at least one of R¹ to R³ is the functional group represented by -O-Si(R⁴)₃ or the functional group represented by -O-Si(R⁵)₂-X¹, the remaining R¹ to R³ may be the same or different and each may be a substituted or unsubstituted monovalent hydrocarbon group. Examples of the unsubstituted monovalent hydrocarbon group as R¹ to R³ include: linear, branched, or cyclic alkyl groups such as methyl, ethyl, n-propyl, i-propyl, n-butyl, i-butyl, s-butyl, pentyl, neopentyl, cyclopentyl, and hexyl; aryl groups such as phenyl, tolyl, and, xylyl groups: and aralkyl groups. Examples of the substituted monovalent hydrocarbon group as R¹ to R³ include: perfluoroalkyl groups such as 3,3,3-trifluoropropyl and 3,3,4,4,4-pentafluorobutyl groups; aminoalkyl groups such as 3-aminopropyl and 3-(aminoethyl)aminopropyl groups; and amidoalkyl groups such as acetylaminoalkyl groups. Moreover, the hydrocarbon group as R¹ to R³ may be partially substituted by a hydroxyl, alkoxy, polyether, or perfluoropolyether group. Examples of the alkoxy group include methoxy, ethoxy, and propoxy groups.

In the formula (1), when one or two of R¹ to R³ are respectively the functional group represented by -O-Si(R⁴)₃ or the functional group represented by -O-Si(R⁵)₂-X¹, the remaining R¹ to R³ are respectively preferably a linear or branched alkyl group having 1 to 6 carbon atoms, particularly preferably a methyl or ethyl group. Particularly, in the formula (1), it is preferred that all or two of R¹ to R³ should respectively be the functional group represented by -O-Si(R⁴)₃ or the functional group represented by -O-Si(R⁵)₂-X¹, and the remaining R¹ to R³ should be a methyl or ethyl group.

Moreover, M is a divalent or trivalent metal atom and wholly or partially forms, as a divalent or trivalent cation, a metal salt with the terminal carboxyl group of the organopolysiloxane derivative represented by the formula (1) in a solid and an aqueous solution. Examples of such a divalent or trivalent metal atom include Zn, Mg, Ca, Ba, Mn, Fe, Co, Al, Ni, Cu, V, Mo, Nb, and Ti. Of these divalent or trivalent metal atoms, particularly, Zn or Mg can be used preferably.

A is a linear or branched alkylene group represented by C_{q}H_{2q}, and q is any integer of 0 to 20. In this context, when q=0, the organosiloxane derivative represented by the formula (1) is a compound represented by the following formula (1-A), wherein the carboxyl-modified group is bound with silicon via an ethylene group. In the present invention, q is preferably any integer of 2 to 15, more preferably any integer of 6 to 12. On the other hand, if q exceeds the upper limit, the resulting gel composition offers poor sebum resistance.

R¹R²R³Si-(CH₂)₂-COOM (1-A)

Moreover, the organosiloxane derivative represented by the formula (1) is characterized by containing a total of 2 to 100 silicon atoms on average per molecule. The organosiloxane derivative represented by the formula (1) contains preferably a total of 3 to 30 silicon atoms on average. On the other hand, if the total number of the silicon atoms per molecule exceeds 100, it may be difficult to adhere to the powders, resulting in poor water resistance as well as poor sebum resistance.

The organosiloxane derivative represented by the formula (1) that can be used preferably is more specifically an organosiloxane derivative wherein R¹ and R² are respectively a functional group represented by -O-Si(R⁴)₃ in which R⁴ is an alkyl group having 1 to 6 carbon atoms; R³ is a linear or branched alkyl group having 1 to 6 carbon atoms; and q is any integer of 6 to 12.

The organosiloxane derivative represented by the formula (1) is obtained by causing addition reaction between polysiloxane containing a silicon-bound hydrogen atom, represented by R¹R²R³SiH and a trimethylsilyl carboxylate derivative having a vinyl end, represented by CH=CH₂-A-COOSiMe₃ in the presence of a platinum-based catalyst, adding, to the reaction product, at least 1 mol or more of a monohydric alcohol (e.g., methanol), water, or a mixture thereof per mol of the trimethylsilyl group as a protective group, and heating the mixture to deprotect the protective group by alcoholysis, followed by a neutralization step using a compound containing a divalent or trivalent metal (M) cation. In this context, R¹, R², R³, and A are as defined above.

In this context, the compound containing a divalent or trivalent metal (M) cation is not particularly limited as long as it is a compound containing a divalent or trivalent metal (M) cation in its structure represented by a composition formula. It is preferred to use a water-soluble divalent or trivalent metal salt for efficiently performing neutralization and ion exchange. Examples thereof can include zinc (Zn²⁺) salts, magnesium (Mg²⁺) salts, calcium (Ca²⁺) salts, barium (Ba²⁺) salts, iron (Fe²⁺ and Fe³⁺) salts, cobalt (Co²⁺) salts, aluminum (Al³⁺) salts, nickel (Ni²⁺) salts, and copper (Cu²⁺) salts. Examples of a counter anion for constituting these metal salts can include, but not limited to, chloride ions (Cl⁻), sulfuric acid ions (SO₄²⁻), nitric acid ions (NO₃⁻), acetic acid ions (CH₃COO⁻), phosphoric acid ions (PO₄³⁻), hydrogen phosphate ions (HPO₄²⁻), and lactic acid ions (CH₃CH(OH)COO⁻). Furthermore, the compound containing a divalent or trivalent metal (M) cation may be a metal salt containing a monovalent alkali metal ion and a divalent or trivalent metal (M) cation, such as potassium aluminum sulfate dodecahydrate (AlK(SO₄)₂·12H₂O).

On the other hand, when the neutralization step using a compound containing a divalent or trivalent metal (M) cation or using a compound containing a monovalent metal ion is not performed after the deprotection, an organosiloxane derivative having a terminal carboxyl group or a monovalent metal salt thereof, represented by the following formula (1'), can be obtained.

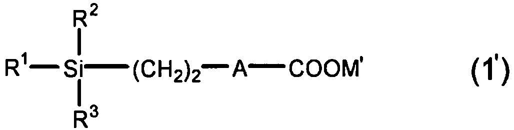

In the formula (1'), R¹ to R³ and A are as defined above; M' is a hydrogen atom or a monovalent metal atom; and the organosiloxane derivative represented by the formula (1') contains a total of 2 to 100 silicon atoms (Si) on average per molecule.

In this context, the terminal carboxyl group of the organosiloxane derivative represented by the formula (1') can be converted easily to a divalent or trivalent metal salt. For example, the organosiloxane derivative represented by the formula (1') is converted to the form of a sodium salt in a 1% aqueous sodium hydroxide solution, and salt exchange can then be performed by the addition of an aqueous solution of a water-soluble zinc salt such as zinc chloride to obtain a zinc salt of the organosiloxane derivative.

For the surface treating agent according to the present invention, the organosiloxane derivative represented by the formula (1) can be obtained easily by mixing the organosiloxane derivative having a terminal carboxyl group or a monovalent metal salt thereof, represented by the formula (1'), with a metal salt solution containing a divalent or trivalent metal ion to cause the cation exchange reaction of the terminal carboxyl group. Examples of such a divalent or trivalent metal salt can include the same metal salts as those exemplified above. Particularly, zinc (Zn²⁺) salts, magnesium (Mg²⁺) salts, calcium (Ca²⁺) salts, and aluminum (Al³⁺) are preferable. A counter anion for constituting these metal salts is preferably chloride ions (Cl⁻), sulfuric acid ions (SO₄²⁻), nitric acid ions (NO³⁻), acetic acid ions (CH₃COO-), phosphoric acid ions (PO₄³⁻), hydrogen phosphate ions (HPO₄²⁻), and lactic acid ions (CH₃CH(OH)COO⁻). Most preferable examples of the divalent or trivalent metal salt include water-soluble zinc salts such as zinc chloride, zinc nitrate, zinc sulfate, and zinc acetate. The amount of the metal salt containing a divalent or trivalent metal ion, used with respect to the organosiloxane derivative having a terminal carboxyl group or a monovalent metal salt thereof, represented by the formula (1'), is preferably an organosiloxane derivative:metal salt mass ratio in the range of 6:1 to 2:1, though the amount is not limited thereto.

Moreover, the method for producing the organosiloxane derivative represented by the formula (1') is described in detail in Japanese Unexamined Patent Publication Nos. 2000-072784, 2000-239390, and 2001-213885. The organosiloxane derivative of the present invention represented by the formula (1') can be produced easily, particularly by a production method comprising the following steps (1) to (3).

### Step (1) :

The step of causing addition reaction between organosilane having a dimethylsiloxy group, represented by

HSi(-O-SiR₂H)_{f}R^{L}_{3-f}

(wherein R is an alkyl group having 1 to 6 carbon atoms or a phenyl group; R^{L} may be the same or different and is a substituted or unsubstituted monovalent hydrocarbon group; and f is any integer of 1 to 3) and a trimethylsilyl carboxylate derivative having a vinyl end, represented by CH=CH₂-A-COOSiMe₃ (wherein A is as defined above) in the presence of a platinum-based transition metal catalyst to obtain an intermediate (1-1) of the following formula:

Si(-O-SiR₂H)_{f}R^{L}_{3-f}-(CH₂)₂-A-COOSiMe₃ (1-1)

### Step (2) :

The step of causing addition reaction between organosilane having an alkenyl group, represented by

R^{B}Si(O-R⁶)ₐᵢ(OSiR⁷R⁸-Xⁱ⁺¹)₃₋ₐᵢ

(wherein R^{B} is a linear or branched alkenyl group represented by CᵣH₂ᵣ; r is any integer of 2 to 20; and R⁶, R⁷, R⁸, Xⁱ⁺¹, and ai are as defined above) and the intermediate (1-1) in the presence of a platinum-based catalyst to obtain an intermediate (1-2) of the following formula:

Si{-O-SiR₂-B-Si(O-R⁶)ₐᵢ(OSiR⁷R⁸-Xⁱ⁺¹)₃₋ₐᵢ}R^{L}_{3-f}-(CH₂)₂-A-COOSiMe₃ (1-2)

### Step (3) :

The step of adding, to the intermediate (1-2), at least 1 mol or more of a monohydric alcohol (e.g., methanol), water, or a mixture thereof per mol of the trimethylsilyl group as a protective group, and heating the mixture to deprotect the protective group by alcoholysis.
In the organosiloxane derivative represented by the formula (1'), when M' is a metal atom, the production method further comprises a neutralization step using the corresponding metal ion (Mₙ⁺). As an example, such a step is the step of neutralizing the carboxyl group (-COOH) by the addition of an aqueous solution of sodium hydroxide (NaOH), potassium hydroxide (KOH), or the like.

Subsequently, the organosiloxane derivative represented by the following formula (3) will be described.

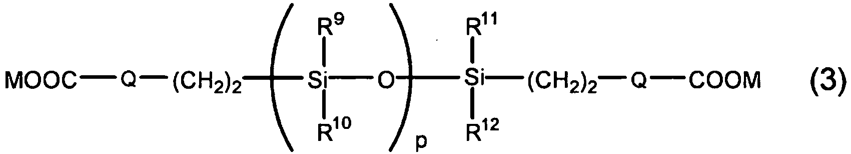

The organosiloxane derivative represented by the formula (3) is an organosiloxane derivative modified, at both ends of the molecular chain, with an alkylcarboxyl group.
In the formula (3), R⁹ to R¹² may be the same or different and are selected from substituted or unsubstituted monovalent hydrocarbon groups. Examples of the unsubstituted monovalent hydrocarbon group represented by R⁹ to R¹² include: linear or branched alkyl groups such as methyl, ethyl, propyl, butyl, pentyl, hexyl, heptyl, octyl, decyl, and dodecyl groups; linear or branched alkenyl groups such as allyl and hexenyl groups; cycloalkyl groups such as cyclopentyl and cyclohexyl groups; aryl groups such as phenyl, tolyl, and naphthyl groups; and aralkyl groups such as benzyl, phenylethyl, phenylpropyl, naphthylmethyl, and naphthylethyl groups. Examples of the substituted monovalent hydrocarbon group represented by R⁹ to R¹² include groups in which hydrogen atoms bound with the carbon atoms of the hydrocarbon groups described above are partially substituted by an organic group such as a hydroxyl group, a halogen atom, an epoxy group, an amino group, a methacryl group, a mercapto group, an alkoxy group, a polyether group, or a perfluoropolyether group and specifically include: perfluoroalkyl groups such as 3,3,3-trifluoropropyl and 3,3,4,4,4-pentafluorobutyl groups; aminoalkyl groups such as 3-aminopropyl and 3-(aminoethyl)aminopropyl groups; and amidoalkyl groups such as acetylaminoalkyl groups. R⁹ to R¹² are respectively preferably an alkyl group having 1 to 20 carbon atoms, an aryl group, or an aralkyl group. It is particularly preferred that 90% by mol or more of R⁹ to R¹² in one molecule should be a methyl group and/or a phenyl group.

Moreover, M is a divalent or trivalent metal atom and wholly or partially forms, as a divalent or trivalent cation, a metal salt with the terminal carboxyl group of the organopolysiloxane derivative represented by the formula (1) in a solid and an aqueous solution. Examples of such a divalent or trivalent metal atom include Zn, Mg, Ca, Ba, Mn, Fe, Co, Al, Ni, Cu, V, Mo, Nb, and Ti. Of these divalent or trivalent metal atoms, particularly, Zn or Mg can be used preferably.

Q is a linear or branched alkylene group represented by C_{q}H_{2q}, and q is any integer of 0 to 20. In this context, when q=0, the organosiloxane derivative represented by the formula (3) is a compound represented by the following formula (3-A), wherein the carboxyl-modified group is bound with silicon via an ethylene group. In the present invention, q is preferably any integer of 6 to 20, more preferably any integer of 6 to 12. On the other hand, if q exceeds the upper limit, the resulting gel composition offers poor sebum resistance. MOOC-(CH₂)₂-(SiR⁹R¹⁰-O)ₚ-SiR¹¹R¹²-(CH₂)₂-COOM (3-A)

In the formula (3), p represents the average degree of polymerization of di-substituted polysiloxane and is any number of 0 to 20. In the present invention, p is more preferably any number of 1 to 20, particularly preferably any number of 1 to 10. On the other hand, if p exceeds the upper limit, it may be difficult to adhere to the powders, resulting in poor water resistance as well as poor sebum resistance.

The organosiloxane derivative represented by the formula (3) that can be used preferably is an organosiloxane derivative wherein R⁹ to R¹² are respectively an alkyl group having 1 to 6 carbon atoms; q is any integer of 0 to 20; and p is any number of 0 to 20.

The organosiloxane derivative represented by the formula (3) is obtained by causing addition reaction between organohydrogenpolysiloxane having a silicon-bound hydrogen atom at both ends of the molecular chain, represented by

H-(SiR⁹R¹⁰-O)ₚ-SiR¹¹R¹²-H

(wherein R⁹ to R¹² are as defined above; and p is any number of 0 to 20) and at least 2 mol of a trimethylsilyl carboxylate derivative having a vinyl end, represented by CH=CH₂-Q-COOSiMe₃ with respect to 1 mol of the organohydrogenpolysiloxane in the presence of a platinum-based catalyst, adding, to the reaction product, at least 1 mol or more of a monohydric alcohol (e.g., methanol), water, or a mixture of these compositions per mol of the trimethylsilyl group as a protective group, and heating the mixture to deprotect the protective group by alcoholysis, followed by a neutralization step using a compound containing a divalent or trivalent metal (M) cation. In this context, Q is as defined above. Examples of the compound containing a divalent or trivalent metal (M) cation can include the same metal salts as those exemplified above.

On the other hand, when the neutralization step using a compound containing a divalent or trivalent metal (M) cation or using a compound containing a monovalent metal ion is not performed after the deprotection, an organosiloxane derivative having a terminal carboxyl group or a monovalent metal salt thereof, represented by the following formula (3'), can be obtained.

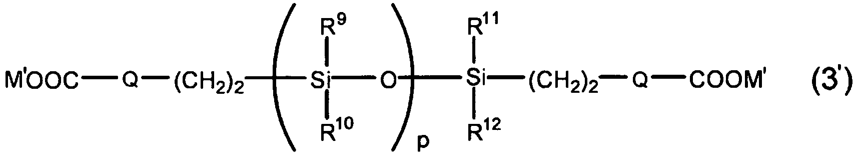

In the formula (3'), R⁹ to R¹² and Q are as defined above; p is as defined above; and M' is a hydrogen atom or a monovalent metal atom.

In this context, the terminal carboxyl group of the organosiloxane derivative represented by the formula (3') can be converted easily to a divalent or trivalent metal salt. For example, the organosiloxane derivative represented by the formula (3') is converted to the form of a sodium salt in a 1% aqueous sodium hydroxide solution, and salt exchange can then be performed by the addition of an aqueous solution of a water-soluble zinc salt such as zinc chloride to obtain a zinc salt of the organosiloxane derivative.

For the surface treating agent according to the present invention, the organosiloxane derivative represented by the formula (3) can be obtained easily by mixing the organosiloxane derivative having a terminal carboxyl group or a monovalent metal salt thereof, represented by the formula (3'), , with a metal salt solution containing a divalent or trivalent metal ion to cause the cation exchange reaction of the terminal carboxyl group. Examples of such a divalent or trivalent metal salt can include the same metal salts as those exemplified above. The amount of the metal salt containing a divalent or trivalent metal ion, used with respect to the organosiloxane derivative having a terminal carboxyl group or a monovalent metal salt thereof, represented by the formula (3'), is preferably an organosiloxane derivative:metal salt mass ratio in the range of 6:1 to 2:1, though the amount is not limited thereto.

The platinum-based catalyst used for producing the organosiloxane derivative of the present invention represented by the formula (1) or (1'), or the formula (3) or (3') is a catalyst for hydrosilylation reaction between the silicon-bound hydrogen atom and the alkenyl group. Examples thereof include chloroplatinic acid, alcohol-modified chloroplatinic acid, olefin complexes of platinum, ketone complexes of platinum, vinylsiloxane complexes of platinum, platinum tetrachloride, fine platinum powders, solid platinum supported by an alumina or silica carrier, platinum black, olefin complexes of platinum, alkenylsiloxane complexes of platinum, carbonyl complexes of platinum, and thermoplastic organic resin (e.g., methyl methacrylate, polycarbonate, polystyrene, and silicone resins) powders containing these platinum-based catalysts. Preferably, the platinum-based catalyst is a 1,3-divinyl-1,1,3,3-tetramethyldisiloxane complex of platinum or chloroplatinic acid.

The organosiloxane derivative represented by the formula (1) or (3) can be used alone as the surface treating agent according to the present invention. In this context, when the moiety M of the organosiloxane derivative is Zn, more specifically, this organosiloxane derivative may have a monomer represented by -COO-Zn⁺X⁻ (X⁻ is a monovalent inorganic or organic anion) or a dimer represented by -COO-Zn-OOC-.

Moreover, the organosiloxane derivative represented by the formula (1') or (3') can be used as the surface treating agent according to the present invention by mixing the organosiloxane derivative with a metal salt solution containing a divalent or trivalent metal ion. Specifically, for the surface treating agent of the present invention, it is required only that the terminal carboxyl group of the organosiloxane derivative can be in a state that forms a divalent or trivalent metal salt when a substance to be treated is treated therewith. Thus, the organosiloxane derivative having a terminal carboxyl group or a monovalent metal salt thereof can be mixed, just before use in surface treatment, with a metal salt solution containing a divalent or trivalent metal ion.

### Surface treating agent

The surface treating agent according to the present invention means a surface treating agent comprising 0.1% by mass or more of the organosiloxane derivative represented by the formula (1) or (3). Particularly, the surface treating agent according to the present invention is preferably a surface treating agent comprising 1.0% by mass or more of a mixture of the organopolysiloxane derivative represented by the formula (1') or (3') and a metal salt solution containing a divalent or trivalent metal ion. Moreover, those having a mass ratio of the organopolysiloxane derivative represented by the formula (1') or (3'):metal salt in the range of 6:1 to 2:1 can be used particularly preferably. In the surface treating agent according to the present invention, the organosiloxane derivative represented by the formula (1) or (3) or the mixture of the organopolysiloxane derivative represented by the formula (1') or (3') and a metal salt solution containing a divalent or trivalent metal ion may be used as a mixture with one or more additional components.

In the surface treating agent according to the present invention, when the organosiloxane derivative represented by the formula (1') or (3') is mixed with a metal salt solution containing a divalent or trivalent metal ion, both the components may be contained in the same preparation or mixed appropriately just before use in treatment. Moreover, the components may be used as a mixture with one or more additional components. The amount of the metal salt solution containing a divalent or trivalent metal ion, used is preferably an organosiloxane derivative:metal salt mass ratio in the range of 6:1 to 2:1.

The surface treating agent according to the present invention can be used in various fields such as medical, cosmetic, fiber, paper, pulp, building material, paint, electric, electronic, automobile, and mechanical fields.

The surface treating agent according to the present invention is particularly preferable for the surface treatment of powders used in cosmetics (including powders and pigments used as coloring agents). The obtained powders treated therewith can have improved texture in use and be excellent in water resistance and sebum resistance. These powders and/or coloring agents that can be used may be any of those used in usual cosmetics, irrespective of their shapes (e.g., spherical, rod-like, needle-like, plate, indefinite, and spindle shapes), classifications based on a particle size range (e.g., fume, fine, and pigment grades), particle structures (e.g., porous and nonporous structures), and the average particle sizes of the powders and/or coloring agents measured using a laser diffraction/scattering particle size distribution measurement method or an electron microscope (in general, the average particle sizes are in the range of 1 to 10,000 nm, though they are not limited to this average particle size range).
Examples of the powders include inorganic powders, organic powders, metal salt powders as surfactants (metallic soaps), colored pigments, pearl pigments, metal powder pigments, silicone resin powders, silicone elastomer powders, polymer powders containing an organic UV protective component, and inorganic UV protective powders. Furthermore, these pigments can be complexed for use.

Examples of the inorganic powders include talc, kaolin, sericite, muscovite, synthetic mica, phlogopite, lepidolite, biotite, lithia mica, vermiculite, calcium carbonate, magnesium carbonate, diatom earth, aluminum silicate, sodium silicate, sodium magnesium silicate, magnesium silicate, magnesium aluminum silicate, calcium silicate, barium silicate, strontium silicate, metal tungstate, silica (silicic acids), hydroxyapatite, zeolite, titanium oxide, iron-doped titanium oxide, zirconium oxide, zinc oxide, cerium oxide, magnesium oxide, barium sulfate, calcium sulfate, magnesium sulfate, higilite, bentonite, montmorillonite, hectorite, ceramics powder, dicalcium phosphate, alumina, boron nitride, ferric hydroxide, and aluminum hydroxide.

Examples of the organic powders include polyester powder, polyethylene powder, polypropylene powder, polymethyl methacrylate powder, polystyrene powder, polyurethane powder, benzoguanamine powder, polymethyl methacrylate powder, polymethyl benzoguanamine powder, polytetrafluoroethylene powder, cellulose, silk powder, nylon powder, 12 nylon powder, 6 nylon powder, styrene/acrylic acid copolymer powder, divinylbenzene/styrene copolymer powder, vinyl resin powder, urea resin powder, phenol resin powder, fluororesin powder, acrylic resin powder, melamine resin powder, epoxy resin powder, polycarbonate resin powder, fine crystal fiber powder, starch powder, and lauroyl lysine powder.

Examples of the metal salt powders as surface active agents include zinc stearate, aluminum stearate, calcium stearate, magnesium stearate, zinc myristate, magnesium myristate, zinc cetyl phosphate, calcium cetyl phosphate, and sodium zinc cetyl phosphate.

Examples of the colored pigments include inorganic red pigments such as iron oxide, ferric hydroxide, and iron titanate; inorganic brown pigments such as gamma-iron oxide; inorganic yellow pigments such as yellow iron oxide and loess; inorganic black pigments such as black iron oxide and carbon black; inorganic purple pigments as mangoviolet and cobaltviolet; inorganic green pigments such as chromium hydroxide, chromium oxide, cobalt oxide, and cobalt titanate; inorganic blue pigments such as Prussian blue and ultramarine; lake tar colorants of Red No.3, Red No.104, Red No.106, Red No. 201, Red No. 202, Red No. 204, Red No.205, Red No.220, Red No.226, Red No.227, Red No.228, Red No.230, Red No.401, Red No.505, Yellow No.4, Yellow No.5, Yellow No. 202, Yellow No.203, Yellow No.204, Yellow No.401, Blue No.1, Blue No.2, Blue No.201, Blue No.404, Green No.3, Green No.201, Green No.204, Green No.205, Orange No.201, Orange No.203, Orange No.204, Orange No.205, Orange No.206, and Orange No.207; and lake natural colorants of chlorophyll, beta-carotene, carminic acid, laccaic acid, carthamin, brazilin, and crocin. Examples of the pearl pigments include titanium oxide-coated mica, titanium oxide-coated mica, bismuth oxychloride, titanium oxide-coated bismuth oxychloride, titanium oxide-coated talc, fish scale flake, and colored titanium oxide-coated mica.

Examples of the metal powder pigments include metal powders such as aluminum powder, gold powder, silver powder, copper powder, platinum powder, and stainless powder.
Examples of the silicone resin powders include MDT and MDQ silicone resin powder, polymethylsilsesquioxane spherical powder, and in particular Torayfil R series from Dow Corning Toray Co., Ltd..

Examples of the silicone elastomer powders include silicone rubber powder and silicone elastomer spherical powder, for example which are described in Japanese Unexamined Patent Publications H2-243612, H8-12545, H8-12546, H8-12524, H9-241511, H10-36219, H11-193331, and 2000-281523, and specific example of such silicone elastomer powder include Torayfil E series (such as Torayfil E-505, 506, 507, and 508) commercially available from Dow Corning Toray Co., Ltd. and cross-linked silicone powder listed in "Japanese Cosmetic Ingredients Codex".

The polymer powders containing an organic UV protective component are polymer powders containing any of UV protective components described later. The polymer powders may or may not be hollow and can have an average primary particle size in the range of 0.1 to 50 µm. Their particle size distributions may be broad or sharp. Examples of the polymer types include the same powder components as those exemplified in the organic powders. Acrylic resins, methacrylic resins, styrene resins, polyurethane resins, polyethylene, polypropylene, polyethylene terephthalate, silicone resins, nylons, and acrylamide resins are preferable. The polymer powders are particularly preferably polymer powders containing the organic UV protective component in the range of 0.1 to 30% by mass. Particularly, polymer powders containing an UV-A absorber 4-tert-butyl-4'-methoxydibenzoylmethane are preferable.

Examples of the above-mentioned organic UV protective components include salicylic acid UV protective components such as homomenthyl salicylate, octyl salicylate, and triethanolamine salicylate; PABA UV protective components such as p-aminobenzoic acid, ethyl dihydroxypropyl PABA, glyceryl PABA, octyl dimethyl PABA, amyl dimethyl PABA, and 2-ethylhexyl dimethyl PABA; benzophenone UV protective components such as 4-(2-beta-glucopyranosiloxy)propoxy-2-hydroxybenzophenone, dihydroxydimethoxybenzophenone, sodium dihydroxydimethoxybenzophenone disulfonate, 2-hydroxy-4-methoxybenzophenone, hydroxymethoxybenzophenone sulfonate and trihydrate thereof, sodium hydroxymethoxybenzophenone sulfonate, 2-hydroxy-4-methoxybenzophenone-5-sulfuric acid, 2,2'-dihydroxy-4-methoxybenzophenone, 2,4-dihydroxybenzophenone, 2,2'4,4'-tetrahydroxybenzophenone, 2,2'-dihydroxy-4,4'-dimethoxybenzophenone, and 2-hydroxy-4-N-octoxybenzophenone; cinnamic acid UV protective components such as 2-ethylhexyl p-methoxycinnamate (also known as octyl paramethoxycinnamate), glyceryl mono-2-ethylhexanoate di-p-methoxycinnamate, 2,5-diisopropyl methyl cinnamate, 2,4,6-tris[4-(2-ethylhexyloxycarbonyl)anilino]-1,3,5-triazine, methyl bis(trimethylsiloxy)silyl isopentyl trimethoxy cinnamate, mixture of isopropyl p-methoxycinnamate and diisopropylcinnamate, and diethanolamine salt p-methoxyhydrocinnamate; benzoylmethane UV protective components such as 2-phenyl-benzimidazole-5-sulfuric acid, 4-isopropyl dibenzoylmethane, and 4-tert-butyl-4'-methoxydibenzoylmethane; 2-cyano-3,3-diphenyl-2-propenoic acid 2-ethylhexyl ester (also known as octocrylene), 2-ethylhexyl dimethoxybenzylidene dioxoimidazolidin propionate, 1-(3,4-dimethoxyphenyl)-4,4-dimethyl-1,3-pentanedione, cinoxate, methyl-o-aminobenzoate, 2-ethylhexyl-2-cyano-3,3-diphenylacrylate, 3-(4-methylbenzylidene)camphor, octyl triaxone, 2-ethylhexyl 4-(3,4-dimethoxyphenyl methylene)-2,5-dioxo-1-imidazolidin propionate, and polymeric derivatives and silane derivatives thereof.

Examples of the inorganic UV protective powders include fine metal oxide particles or fine metal hydroxide particles having an average particle size in the range of 1 to 100 nm, particularly, titanium oxide, zinc oxide, plate iron oxide, aluminum flaks, and ceramics such as silicon carbide.

These powders may undergo, independently of the surface treatment of the present invention, surface treatment conventionally known in the art, for example, fluorine compound treatment (preferably, perfluoroalkyl phosphate, perfluoroalkylsilane, perfluoropolyether, fluorosilicone, and silicone fluoride resin treatments), silicone treatment (preferably, methylhydrogenpolysiloxane, dimethylpolysiloxane, and gas-phase tetramethyltetrahydrogencyclotetrasiloxane treatments), silicone resin treatment (preferably, trimethylsiloxysilicate treatment), pendant treatment (a method comprising adding an alkyl chain, etc., after gas-phase silicone treatment), treatment with silane coupling agents, treatment with titanium coupling agents, silane treatment (preferably alkylsilane and alkylsilazane treatments), oil solution treatment, N-acylated lysine treatment, polyacrylic acid treatment, metallic soap treatment (preferably, using stearic acid or myristate), acrylic resin treatment, and metal oxide treatment, or a combination of two or more of these treatments.
For example, the surface of fine titanium oxide particles is coated with metal oxide such as silicon oxide or alumina, and surface treatment with alkylsilane is then performed. During this process or formulation into a preparation, the surface treatment according to the present invention may be performed.

Specifically, combined use of the surface treatment with the organosiloxane derivative according to the present invention and the surface treatment conventionally known in the art can produce surface-treated powders having both the effects of the present invention and the surface treatment conventionally known in the art.

Moreover, in the present invention, one of these powders or a mixture of two or more thereof may be surface-treated with the organosiloxane derivative represented by the formula (1) or (3) or with the mixture of the organopolysiloxane derivative represented by the formula (1') or (3') and a metal salt solution containing a divalent or trivalent metal ion.

### Surface treatment method

For surface-treating powders with the surface treating agent according to the present invention, the treatment method can be performed, for example, by two processes, dry and wet processes as described below. However, the present invention is not intended to be limited to them.

### Treatment method 1: dry process

An organosiloxane derivative as a compound 1 described below is converted to the form of a sodium salt in a 1% aqueous sodium hydroxide solution, and further, salt exchange is then performed by the addition of a 1% aqueous zinc chloride solution to prepare a Zn salt of the organosiloxane derivative. Then, 15 g of talc and 5 g of the Zn salt of the organosiloxane derivative are pulverized and mixed.

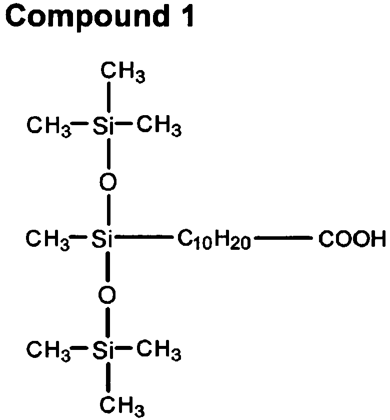

### Treatment method 2: wet process

100 g of talc, 30.5 g of a 1% aqueous sodium hydroxide solution, 3 g of the organosiloxane derivative as the compound 1, and 156 ml of a 1% aqueous zinc chloride solution are sequentially added to prepare a dispersed mixture. This reaction is performed at 70°C. Then, surface-treated powders are isolated by filtration, then washed with water, and dried (105°C, 12 hr). The resulting powders are pulverized using a pulverizer.

For surface-treating powders with the surface treating agent according to the present invention, surface treatment based on the wet process is adopted more preferably. In the wet process, the mixing temperature is preferably 60 to 80°C. Moreover, the mixing time is usually preferably 1 to 3 hours, though it differs depending on the amount of the surface treating agent and the type of the powders.

For surface-treating powders with the surface treating agent according to the present invention, the amount of the surface treating agent is usually preferably 1 to 10% by mass, more preferably 3 to 8% by mass, with respect to untreated powders, though it differs depending on the type of the powders. If the amount is less than 1% by mass, the resulting powders do not sufficiently offer the effect of improving texture in use, when incorporated in cosmetics. Moreover, if the powders are treated with more than 10% by mass of the surface treating agent, the resulting powders tend to deteriorate texture in use, such as poor spreadability, when incorporated in cosmetics. Therefore, such an amount is not preferable.

A surface-treated powder that has improved texture in use and is excellent in water resistance and sebum resistance is obtained by surface-treating a powder with the surface treating agent according to the present invention. Thus, the obtained surface-treated powder, when incorporated in cosmetics, can not only offer spreadability over the skin and adhesion to the skin but exhibit very excellent long-lasting finish effects. Examples of the cosmetics that can be formulated with the surface-treated powder according to the present invention include: makeup cosmetics such as foundations, eye shadows, eyebrow powders, and blushes; body-care cosmetics such as body powders and baby powders; and milky lotions and lotions.

Moreover, when the surface-treated powder according to the present invention is incorporated in a cosmetic, the amount of the surface-treated powder incorporated is preferably 2 to 80% by mass in the cosmetic composition, though it is appropriately determined according to various cosmetics.

### EXAMPLES

The structures of organosiloxane derivatives (compounds 1 to 4 and comparative compounds 1 to 4) used in Examples and Comparative Examples are shown below.

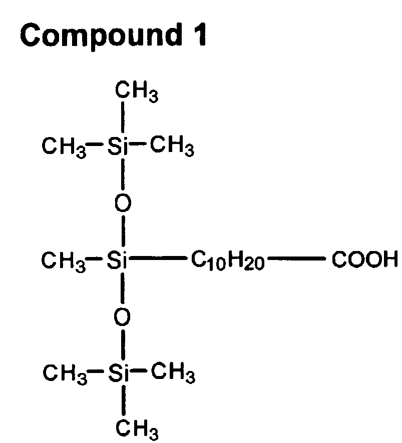

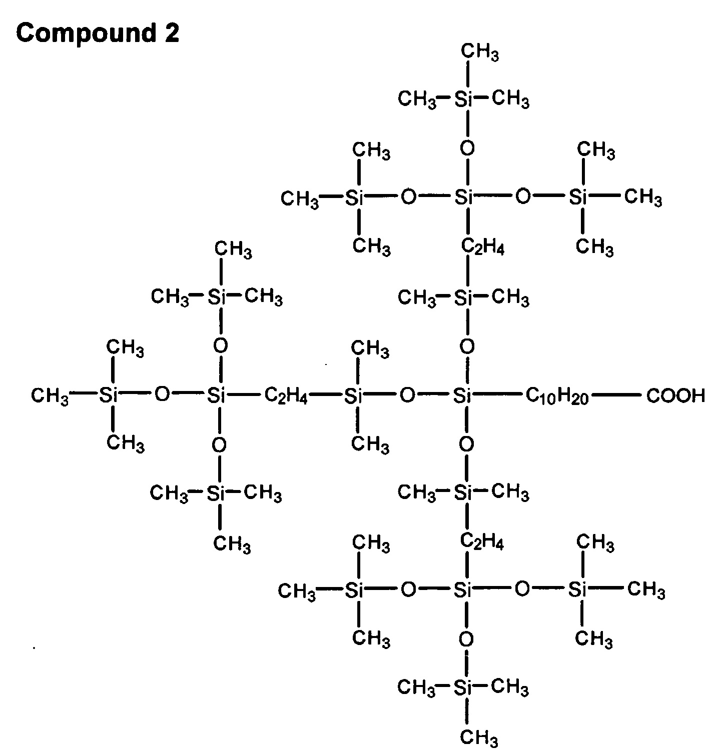

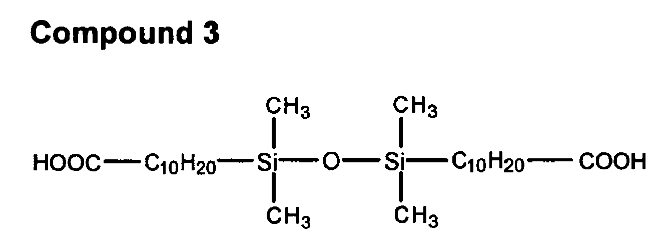

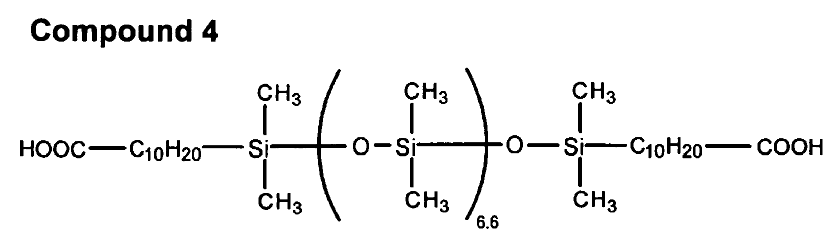

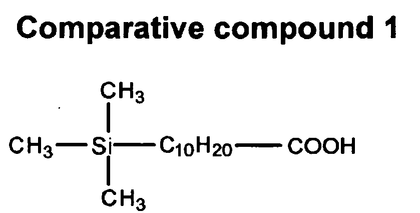

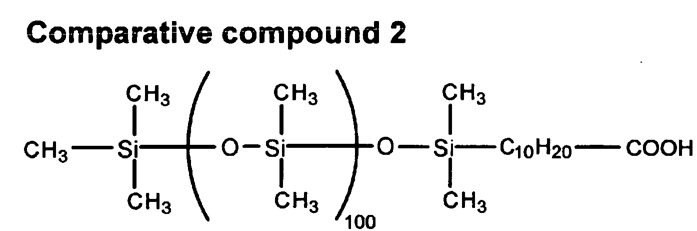

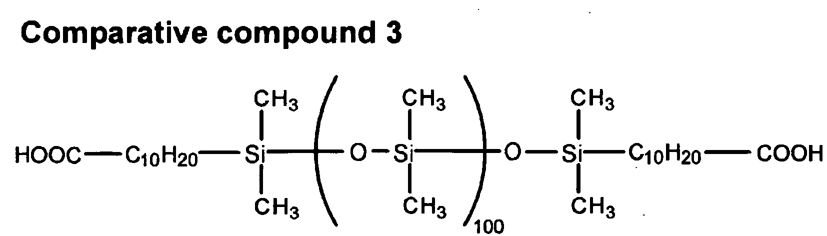

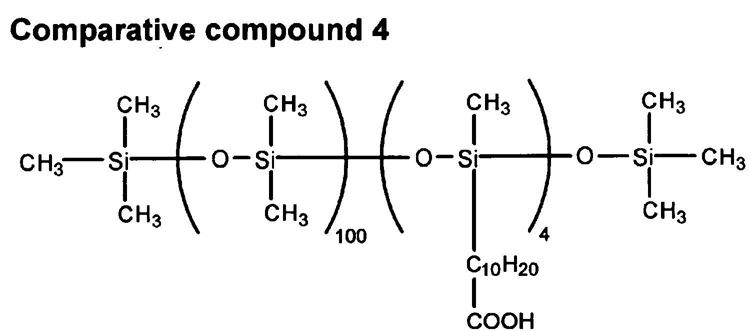

First, the present inventors used a Zn salt of the compound 1 as an organosiloxane derivative and surface-treated talc (trade name: JA68R, manufactured by Asada Milling Co., Ltd.) therewith to prepare a surface-treated powder. The obtained surface-treated powder (Example 1) and commercially available various powders for cosmetics (Comparative Examples 1 to 4) were evaluated for water resistance, sebum resistance, and texture in use (smoothness and fit). The details of the test and evaluation criteria are as shown below. The results are shown in Table 1 below.

### Example 1 (compound 1-Zn salt-treated talc)

100 g of talc, 22.9 g of a 1% aqueous sodium hydroxide solution, and 1 L of water were mixed and dispersed using a propeller. Then, 3 g of the organosiloxane derivative as the compound 1 and 117 ml of a 1% aqueous zinc chloride solution were sequentially added thereto to prepare a dispersed mixture. Then, surface-treated powders were isolated by filtration, then washed with water, and dried (105°C, 12 hr). The resulting powders were pulverized using a pulverizer to obtain a surface-treated powder of Example 1 (the amount of the surface treating agent: 3.0% by mass).

### Comparative Examples 1 to 4 (commercially available powders for cosmetics)

Commercially available powders for cosmetics used as Comparative Examples are as follows:
Comparative Example 1: dimethicone-treated talc (SA Talc JA-68R: manufactured by Miyoshi Kasei Inc., the amount of the surface treating agent: 2.0% by mass)
Comparative Example 2: silicone-treated talc (BAE Talc JA-68R: manufactured by Miyoshi Kasei Inc., the amount of the surface treating agent: 2.0% by mass) Comparative Example 3: Ca stearate-treated talc (Talc ACS-515: manufactured by Fujimoto Chemicals Co., Ltd., the amount of the surface treating agent: 5.0% by mass)
Comparative Example 4: Mg myristate-treated barium sulfate (H-LFM: manufactured by Sakai Chemical Industry Co., Ltd., the amount of the surface treating agent: 1.0% by mass)

### Evaluation (1): water resistance

### (Test method)

The angle of contact was measured using FACE automatic contact angle meter (CA-V150 model, manufactured by Kyowa Interface Science Co., Ltd.). The measurement was performed according to a sessile drop method using purified water. A larger numeric value means more excellent water resistance.

### (Evaluation criteria)

O: 110° or larger
Δ: 105° or larger and smaller than 110°
X: smaller than 105°

### Evaluation (2): sebum resistance

### (Test method)

The angle of contact was measured using FACE automatic contact angle meter (CA-V150 model, manufactured by Kyowa Interface Science Co., Ltd.). The measurement was performed according to a sessile drop method using squalene. A larger numeric value means more excellent sebum resistance.

### (Evaluation criteria)

O: 45° or larger
Δ: 40° or larger and smaller than 45°
X: smaller than 40°

### Evaluation (3): smoothness and lubricity

### (Test method)

The coefficient of kinetic friction was measured using Tribogear (manufactured by Shinto Scientific Co., Ltd.).
Measurement method: the powder was applied onto a double-faced tape, and the measurement was performed using a 1 cmφ stainless cylinder as a contactor. A smaller numeric value means more excellent smoothness.
Based on the obtained coefficient of kinetic friction, the evaluation was conducted on the following grade of 3:
O: a coefficient of kinetic friction smaller than 0.16
Δ: a coefficient of kinetic friction of 0.16 or larger and smaller than 0.20
X: a coefficient of kinetic friction of 0.20 or larger

### Evaluation (4): fit with skin moisturization

Twenty expert panelists evaluated the powder for its fit with skin moisturization. In this context, the evaluation was conducted according to the following criteria (on the grade of 5), and the average thereof is described.

### (Evaluation criteria)

5: the powder was judged as having good skin moisturization.
4: the powder was judged as slightly fitting the skin with skin moisturization.
3: the powder was judged as being average.
2: the powder was judged as barely fitting the skin.
1: the powder was judged as not fitting the skin.
Based on the average, the evaluation is indicated on the following grade of 4:
⊚: an average of 4.5 or higher
O: an average of 3.5 or higher and lower than 4.5
Δ: an average of 2.5 or higher and lower than 3.5
X: an average lower than 2.5

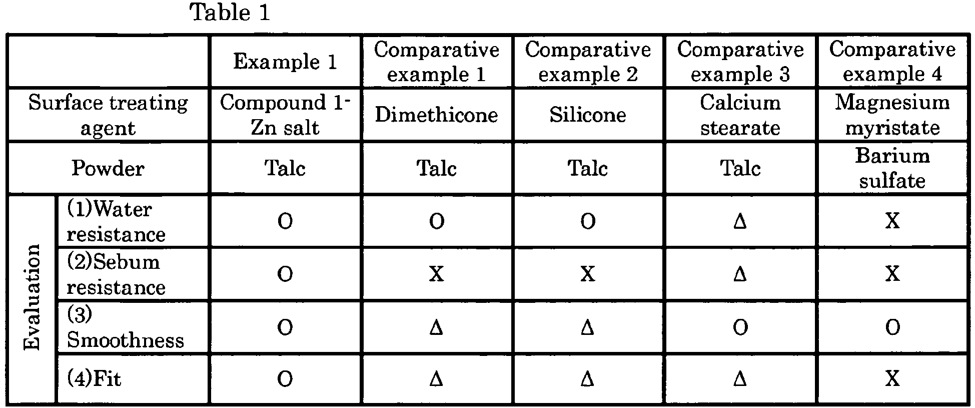

As shown in the Table 1, Example 1 using, as a surface treating agent, the Zn salt of the organosiloxane derivative having an alkylcarboxyl group substituted for the side chain of trisiloxane was excellent in both water resistance and sebum resistance. By contrast, Comparative Examples 1 to 4 using the commercially available surface-treated powders for cosmetics did not exhibited excellent effects in terms of both of water resistance and sebum resistance. Moreover, the surface-treated powder of Test Example 1 that was surface-treated with the Zn salt of the organosiloxane derivative also had very good texture in use such as smoothness and fit and can therefore be expected to have the advantage of being excellent in texture in use when incorporated in cosmetics or the like.

Subsequently, to more closely study the organosiloxane derivative used as a surface treating agent, the present inventors prepared surface-treated powders in the same way as above using the compound 1 (untreated) and an Na salt of the compound 1. The obtained various surface-treated powders were evaluated for water resistance, sebum resistance, and texture in use (smoothness and fit). The details of the test are as shown below. The results are shown in Table 2 below.

### Comparative Example 5 (compound 1 (untreated carboxylic acid) -treated talc)

100 g of talc was mixed in 1 L of water and dispersed using a propeller. Then, 3 g of the organosiloxane derivative as the compound 1 was added thereto to prepare a dispersed mixture. Then, surface-treated powders were isolated by filtration, then washed with water, and dried (105°C, 12 hr). The resulting powders were pulverized using a pulverizer.

### Comparative Example 6 (compound 1-Na salt-treated talc)

100 g of talc, 22.9 g of a 1% aqueous sodium hydroxide solution, and 1 L of water were mixed and dispersed using a propeller. Then, 3 g of the organosiloxane derivative as the compound 1 was added thereto to prepare a dispersed mixture. Then, surface-treated powders were isolated by filtration, then washed with water, and dried (105°C, 12 hr). The resulting powders were pulverized using a pulverizer.

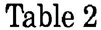

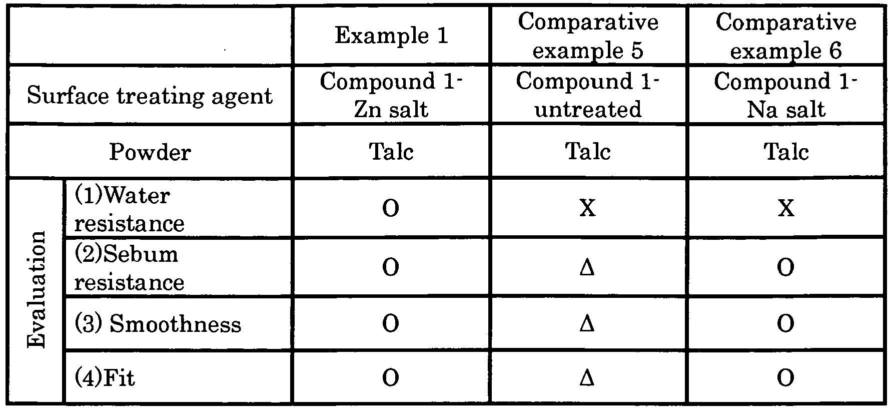

As shown in the Table 2, Example 1 using the organosiloxane derivative having a terminal carboxyl group in the form of a Zn salt was excellent in both water resistance and sebum resistance. By contrast, Comparative Example 1 using the compound 1 in the form of untreated carboxylic acid as well as Comparative Example 2 using the organosiloxane derivative having a carboxyl group in the form of an Na salt did not produce sufficient effects, particularly in terms of water resistance. These results demonstrated that when the organosiloxane derivative having a carboxyl group is used as a surface treating agent, excellent water resistance and sebum resistance are obtained particularly by using the terminal carboxyl group in the form of a Zn salt.

Furthermore, the present inventors prepared surface-treated powders in the same way as in the Example 1 using Zn salts of the organosiloxane derivatives as the compounds 2 to 4 and the comparative compounds 1 to 4. The obtained various surface-treated powders were evaluated for water resistance, sebum resistance, and texture in use. The results are shown in Tables 3 and 4 below.

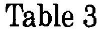

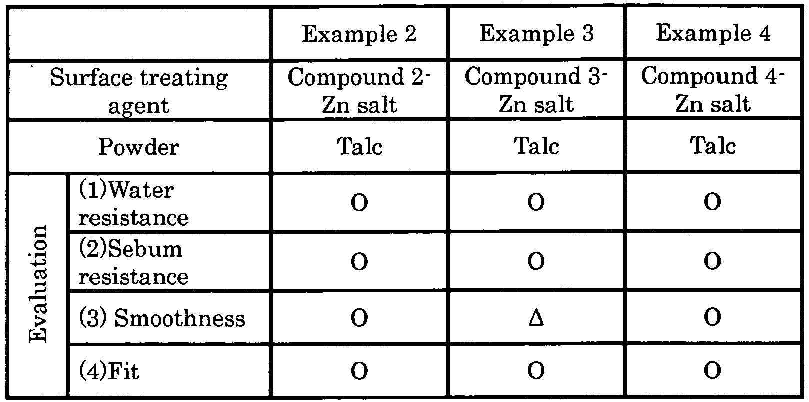

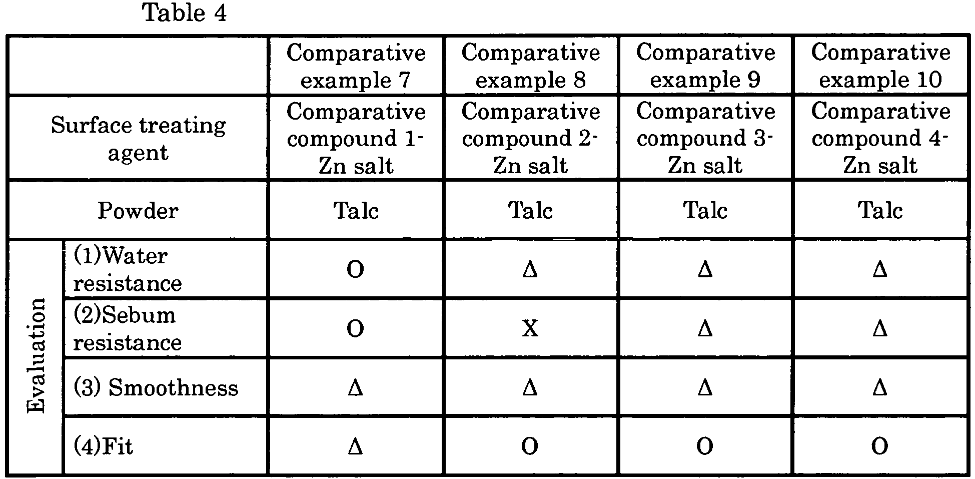

As shown in the Table 3, the Zn salt of the compound 2 having an alkylcarboxyl group substituted for the side chain of dendrimer-type polysiloxane as well as the Zn salts of the compounds 3 and 4 having an alkylcarboxyl group substituted for both ends of linear disiloxane or polysiloxane, when used as surface treating agents, were demonstrated to give surface-treated powders that are excellent in texture in use such as smoothness and fit and excellent in water resistance and sebum resistance.

On the other hand, as shown in the Table 4, the Zn salt of the comparative compound 1 having an alkylcarboxyl group substituted for trimethylsilane as well as the Zn salts of the comparative compounds 2, 3, and 4 having an alkylcarboxyl group substituted for either end, both ends, or side chain of long-chain polysiloxane (Si=100), when used as surface treating agents, could not give surface-treated powders excellent in all of texture in use, water resistance, and sebum resistance.

Moreover, the present inventors prepared surface-treated powders in the same way as in the Example 1 using various metal salts (Zn, Mg, Ca, and Al salts) of the organosiloxane derivative. The obtained various surface-treated powders were evaluated for water resistance, sebum resistance, and texture in use. The results are shown in Table 5 below.

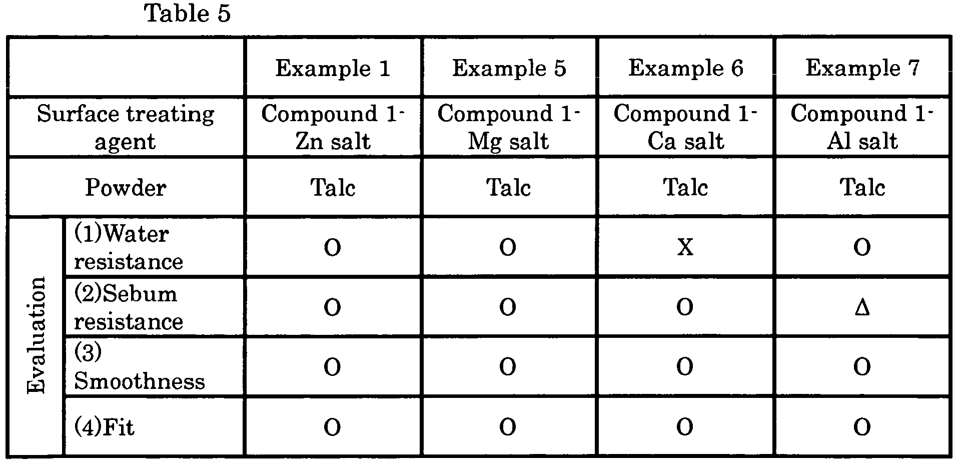

As shown in the Table 5, when varying types of metals were bound to the carboxyl group of the organosiloxane derivative, it was demonstrated the Zn and Mg salts offer both excellent water resistance and sebum resistance and provide fit combined with smoothness in terms of texture in use. By contrast, both the Ca and Al salts produced favorable results in terms of texture in use, although the Ca salt exhibited poor water resistance and the Al salt exhibited slightly poor sebum resistance.

Subsequently, to study a preferable amount of the organosiloxane derivative used as a surface treating agent, the present inventors prepared surface-treated powders in the same way as in the Example 1 by variously changing the amount of the surface treating agent. The obtained various surface-treated powders were evaluated for water resistance, sebum resistance, and texture in use (smoothness and fit). The details of the test and evaluation criteria are the same as those in the test described above. The results are shown in Table 6 below.

### Example 8 (compound 1-Zn salt-treated talc) treatment with 1% surface treating agent

100 g of talc, 7.6 g of a 1% aqueous sodium hydroxide solution, and 1 L of water were mixed and dispersed using a propeller. Then, 1 g of the organosiloxane derivative as the compound 1 and 39 ml of a 1% aqueous zinc chloride solution were sequentially added thereto to prepare a dispersed mixture. Then, surface-treated powders were isolated by filtration, then washed with water, and dried (105°C, 12 hr). The resulting powders were pulverized using a pulverizer.

### Example 9 (compound 1-Zn salt-treated talc) treatment with 6% surface treating agent

100 g of talc, 45.8 g of a 1% aqueous sodium hydroxide solution, and 1 L of water were mixed and dispersed using apropeller. Then, 6 g of the organosiloxane derivative as the compound 1 and 234 ml of a 1% aqueous zinc chloride solution were sequentially added thereto to prepare a dispersed mixture. Then, surface-treated powders were isolated by filtration, then washed with water, and dried (105°C, 12 hr). The resulting powders were pulverized using a pulverizer.

### Example 10 (compound 1-Zn salt-treated talc) treatment with 12% surface treating agent

100 g of talc, 91.6 g of a 1% aqueous sodium hydroxide solution, and 1 L of water were mixed and dispersed using a propeller. Then, 12 g of the organosiloxane derivative as the compound 1 and 468 ml of a 1% aqueous zinc chloride solution were sequentially added thereto to prepare a dispersed mixture. Then, surface-treated powders were isolated by filtration, then washed with water, and dried (105°C, 12 hr). The resulting powders were pulverized using a pulverizer.

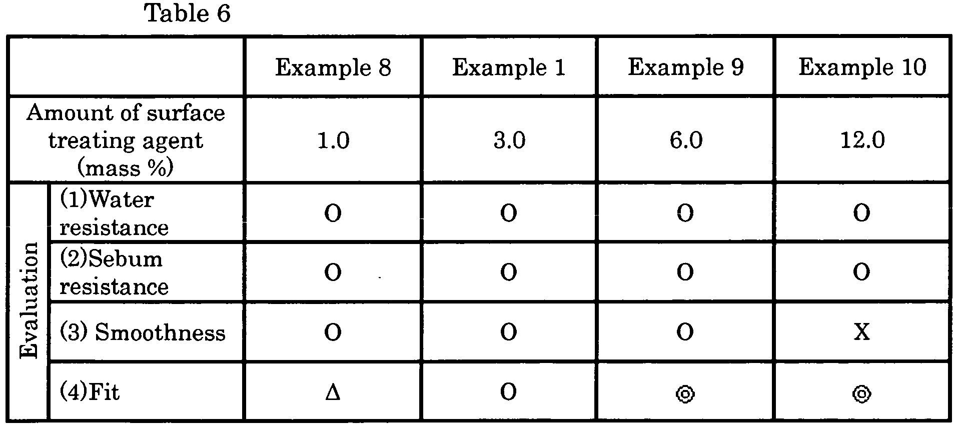

As shown in the Table 6, Examples 1 and 9 using 3.0 or 6.0% by mass of the Zn salt of the organosiloxane derivative in the surface treatment produced the powders that were excellent in both water resistance and sebum resistance and also had smoothness and fit in terms of texture in use. Particularly, Example 9 using 6.0% by mass thereof was demonstrated to be very excellent in fit. On the other hand, Example 8 using the amount of the surface treating agent of 1.0% by mass had slightly poorer fit. Moreover, Example 12 using 12.0% by mass of the surface treating agent was insufficient in terms of smoothness due to poor spreadability, although it was excellent in fit.

Subsequently, the present inventors formulated with the surface-treated powder that was treated with the Zn salt of the specifically structured organosiloxane derivative in cosmetics (powdery foundations). The cosmetics were evaluated for their texture in use (smoothness, fit, and skin moisturization). The composition and evaluation results of various cosmetics used in the test are together shown in Tables 6 and 7 below. In this context, the evaluation was conducted according to criteria shown below.

### Texture in use (smoothness, fit, and skin moisturization)

Ten expert panelists actually applied the cosmetic of each Test Example to their skins and evaluated the texture in use (smoothness upon application, fit upon application, and skin moisturization after application) of the cosmetic. The evaluation was conducted according to the following criteria:
⊚: of ten expert panelists, nine or more judged the cosmetic as being good.
O: of ten expert panelists, six to eight judged the cosmetic as being good.
Δ: of ten expert panelists, three to five judged the cosmetic as being good.
X: of ten expert panelists, two or less judged the cosmetic as being good.

**Table 7**

| Powder foundation | | Example 11 | Example 12 | Example 13 | Example 14 | Example 15 |
|---|---|---|---|---|---|---|
| Alpha-olefin oligomer | | 2.5 | 2.5 | 2.5 | 2.5 | 2.5 |
| Methyl phenyl silicone | | 2 | 2 | 2 | 2 | 2 |
| Diisostearyl malate | | 3 | 3 | 3 | 3 | 3 |
| Sorbitan sesquiisostearate | | 1.5 | 1.5 | 1.5 | 1.5 | 1.5 |
| Hydrophobized yellow iron oxide | | 1.83 | 1.83 | 1.83 | 1.83 | 1.83 |
| Hydrophobized red iron oxide | | 0.66 | 0.66 | 0.66 | 0.66 | 0.66 |
| Hydrophobized black iron oxide | | 0.12 | 0.12 | 0.12 | 0.12 | 0.12 |
| Zinc oxide | | 3 | 3 | 3 | 3 | 3 |
| Mica | | 17 | 17 | 17 | 17 | 17 |
| Synthetic phlogopite | | 15 | 15 | 15 | 15 | 15 |
| Silicone powder | | 8 | 8 | 8 | 8 | 8 |
| Silicone composite powder | | 4 | 4 | 4 | 4 | 4 |
| Compound 1 - Zn salt-treated talc | | 29.17 | - | - | - | 14 |
| Compound 2 - Zn salt-treated talc | | - | 29.17 | - | - | 15.17 |
| Compound 3 - Zn salt-treated talc | | - | - | 29.17 | - | - |
| Compound 4 - Zn salt-treated talc | | - | - | - | 29.17 | - |
| Talc (untreated) | | - | - | - | - | - |
| Comparative compound 1 - Zn salt-treated talc | | - | - | - | - | - |
| Comparative compound 2 - Zn salt-treated talc | | - | - | - | - | - |
| Comparative compound 3 - Zn salt-treated talc | | - | - | - | - | - |
| Comparative compound 4 - Zn salt-treated talc | | - | - | - | - | - |
| Tocopherol | | 0.02 | 0.02 | 0.02 | 0.02 | 0.02 |
| Ethyl paraben | | 0.2 | 0.2 | 0.2 | 0.2 | 0.2 |
| Ethylhexyl methoxycinnamate | | 1.0 | 1.0 | 1.0 | 1.0 | 1.0 |
| Hydrophobized zinc oxide | | 11 | 11 | 11 | 11 | 11 |
| Texture in use | Smoothness | ○ | ○ | Δ | Δ | ○ |
| | Fit | ○ | ○ | ○ | ○ | ○ |
| | skin moisturization | ○ | ○ | ○ | ○ | ○ |

**Table 8**

| Powder foundation | | Comparative example 11 | Comparative example 12 | Comparative example 13 | Comparative example 14 | Comparative example 15 |
|---|---|---|---|---|---|---|
| Alpha-olefin oligomer | | 2.5 | 2.5 | 2.5 | 2.5 | 2.5 |
| Methyl phenyl silicone | | 2 | 2 | 2 | 2 | 2 |
| Diisostearyl malate | | 3 | 3 | 3 | 3 | 3 |
| Sorbitan sesquiisostearate | | 1.5 | 1.5 | 1.5 | 1.5 | 1.5 |
| Hydrophobized yellow iron oxide | | 1.83 | 1.83 | 1.83 | 1.83 | 1.83 |
| Hydrophobized red iron oxide | | 0.66 | 0.66 | 0.66 | 0.66 | 0.66 |
| Hydrophobized black iron oxide | | 0.12 | 0.12 | 0.12 | 0.12 | 0.12 |
| Zinc oxide | | 3 | 3 | 3 | 3 | 3 |
| Mica | | 10 | 10 | 10 | 10 | 10 |
| Titanium oxide | | 1 | 1 | 1 | 1 | 1 |
| Synthetic phlogopite | | 15 | 15 | 15 | 15 | 15 |
| Silicone powder | | 8 | 8 | 8 | 8 | 8 |
| Silicone composite powder | | 4 | 4 | 4 | 4 | 4 |
| Compound 1 - Zn salt-treated talc | | - | - | - | - | - |
| Compound 2 - Zn salt-treated talc | | - | - | - | - | - |
| Compound 3 - Zn salt-treated talc | | - | - | - | - | - |
| Compound 4 - Zn salt-treated talc | | - | - | - | - | - |
| Talc (untreated) | | 29.17 | - | - | - | - |
| Comparative compound 1 - Zn salt-treated talc | | - | 29.17 | - | - | - |
| Comparative compound 2 - Zn salt-treated talc | | - | - | 29.17 | - | - |
| Comparative compound 3 - Zn salt-treated talc | | - | - | - | 29.17 | - |
| Comparative compound 4 - Zn salt-treated talc | | - | - | - | - | 29.17 |
| Tocopherol | | 0.02 | 0.02 | 0.02 | 0.02 | 0.02 |
| Ethyl paraben | | 0.2 | 0.2 | 0.2 | 0.2 | 0.2 |
| Ethylhexyl methoxycinnamate | | 1.0 | 1.0 | 1.0 | 1.0 | 1.0 |
| Hydrophobized zinc oxide | | 11 | 11 | 11 | 11 | 11 |
| Texture in use | Smoothness | Δ | Δ | X | X | X |
| | Fit | Δ | Δ | ○ | ○ | ○ |
| | skin moisturization | Δ | Δ | ○ | ○ | ○ |

As shown in the Table 6, the powdery foundations of Examples 11 to 15 formulated with the surface-treated talc that was treated with the organosiloxane derivative-Zn salt as any of the compounds 1 to 4 were excellent in texture in use in terms of all of smoothness, fit, and skin moisturization.

By contrast, as shown in the Table 7, the powdery foundation of Comparative Example 11 formulated with the untreated talc as well as the powdery foundations of Comparative Examples 12 to 15 formulated with the surface-treated talc that was treated with the Zn salt of any of the comparative compounds 1 to 4 were less than satisfactory, particularly in terms of smoothness.

Hereinafter, Formulation Examples of the cosmetic of the present invention formulated with the surface-treated powder that is treated with the surface treating agent of the present invention will be shown specifically. However, the present invention is not intended to be limited to them.

### Formulation example 1: foundation

| (Components) | (mass %) |
|---|---|
| (1) Compound 1 - Zn salt-treated talc | 30 |
| (2) Mica | 26.85 |
| (3) Zinc oxide | 3 |
| (4) Fine titanium oxide | 1 |
| (5) Hydrophobized titanium oxide | 11 |
| (6) Hydrophobized yellow iron oxide | 2.7 |
| (7) Hydrophobized red iron oxide | 1 |
| (8) Hydrophobized black iron oxide | 0.25 |
| (9) Barium sulfate | 6 |
| (10) Cross-linked dimethicone copolymer | 8 |
| (11) Isooctyl methoxycinnamate | 1 |
| (12) Hydrogenated olefin oligomer | 2.5 |
| (13) Methylphenylpolysiloxane | 2 |
| (14) Diisostearyl malate | 3 |
| (15) Sorbitan sesquiisostearate | 1.5 |
| (16) Paraben | 0.2 |

### (Production method)

The components (1) to (10) and (16) were mixed and pulverized. Then, the oil components (11) to (15) heated to 80°C were added thereto and mixed. The obtained powder was molded to obtain the foundation of interest.

### Formulation example 2: loose powder

| (Components) | (mass %) |
|---|---|
| (1) Compound 3 - Zn salt-treated sericite | 20 |
| (2) Hydrophobized talc | 68 |
| (3) Zinc oxide | 1 |
| (4) Zinc myristate | 4 |
| (5) Crosslinked silicone/network silicone block copolymer (Production method) | 7 |

The components (1) to (5) were mixed and pulverized to obtain the loose powder of interest.

### Formulation example 3: face powder

| (Components) | (mass %) |
|---|---|
| (1) Compound 2 - Zn salt-treated talc | 50 |
| (2) Talc | 23.22 |
| (3) Mica | 3 |
| (4) Zinc oxide | 1 |
| (5) Titanated mica | 13 |
| (6) Yellow iron oxide | 0.5 |
| (7) Red iron oxide | 0.07 |
| (8) Black iron oxide | 0.01 |
| (9) Polymethyl methacrylate | 1 |
| (10) Petrolatum | 1 |
| (11) Synthetic hydrocarbon wax | 2 |
| (12) Glyceride triisooctanoate | 5 |
| (13) Paraben | 0.2 |

### (Production method)

The components (1) to (9) and (13) were mixed and pulverized. Then, the oil components (10) to (12) heated to 80°C were added thereto and mixed. The obtained powder was molded to obtain the face powder of interest.

### Formulation example 4: liquid foundation

| (Components) | (mass %) |
|---|---|
| (1) Decamethylcyclopentasiloxane | 30 |
| (2) Dimethylpolysiloxane | 5 |
| (3) Polyether-modified silicone | 3 |
| (4) Organic-modified bentonite | 1.5 |
| (5) Polymethyl metacrylate spherical powder | 5 |
| (6) Hydrophobized yellow iron oxide | 2 |
| (7) Hydrophobized red iron oxide | 1 |
| (8) Hydrophobized black iron oxide | 0.2 |
| (9) Compound 1 - Zn salt-treated titanium oxide | 8 |
| (10) Ion-exchange water | 36.8 |
| (11) Glycerin | 2 |
| (12) 1,3-Butylene glycol | 5 |
| (13) Phenoxyethanol | 0.5 |

### (Production method)

The components (1) to (4) were mixed and dispersed. The components (5) to (9) were added thereto to prepare a dispersed mixture. Then, an aqueous phase of the components (10) to (13) was added to the mixture for emulsification to obtain the liquid foundation of interest.

### Formulation example 5: pre-makeup for cosmetics

| (Components) | (mass %) |
|---|---|
| (1) Decamethylcyclopentasiloxane | 30 |
| (2) Dimethylpolysiloxane | 5 |
| (3) Polyether-modified silicone | 3 |
| (4) Organic-modified bentonite | 1.5 |
| (5) Polymethyl metacrylate spherical powder | 5 |
| (6) Hydrophobized titanium | 2 |
| (7) Organopolysiloxane elastomer spherical powder | 5 |
| (8) Hydrophobized black iron oxide | 0.2 |
| (9) Compound 1 - Mg salt-treated talc | 5 |
| (10) Ion-exchange water | 35.8 |
| (11) Glycerin | 2 |
| (12) 1,3-Butylene glycol | 5 |
| (13) Phenoxyethanol | 0.5 |

### (Production method)

The components (1) to (4) were mixed and dispersed. The components (5) to (9) were added thereto to prepare a dispersed mixture. Then, an aqueous phase of the components (10) to (13) was added to the mixture for emulsification to obtain the makeup base of interest.

## Claims

1. A surface treating method comprising; treating a surface of a powder using a composition_comprising an organosiloxane derivative having a divalent or trivalent metal salt of a terminal carboxyl group, represented by the following formula (1) or (3); 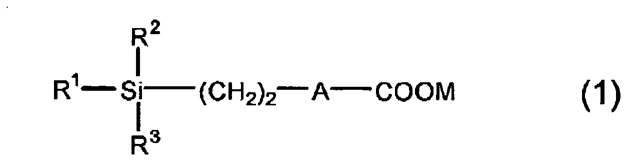 in the formula (1), at least one of R¹ to R³ is a functional group represented by -O-Si(R⁴)₃ in which R⁴ is an alkyl group having 1 to 6 carbon atoms or a phenyl group, or a functional group represented by -O-Si(R⁵)₂-X¹ in which R⁵ is an alkyl group having 1 to 6 carbon atoms or a phenyl group, and X¹ is a functional group represented by the following formula (2) when i=1; and the remaining R¹ to R³ may be the same or different and each may be a substituted or unsubstituted monovalent hydrocarbon group; M is a divalent or trivalent metal atom; A is a linear or branched alkylene group represented by C_{q}H_{2q} in which q is any integer of 0 to 20; and the organosiloxane derivative represented by the formula (1) contains a total of 2 to 100 silicon atoms (Si) on average per molecule; 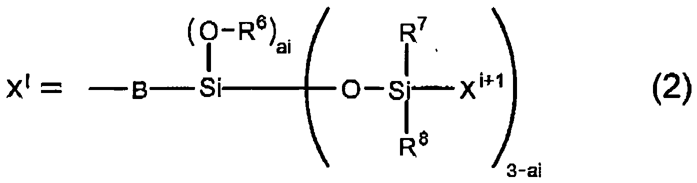 in the formula (2), R⁶ is a hydrogen atom, an alkyl group having 1 to 6 carbon atoms, or a phenyl group; R⁷ and R⁸ are respectively an alkyl group having 1 to 6 carbon atoms or a phenyl group; B is a linear or branched alkylene group represented by CᵣH₂ᵣ which may be partially branched in which r is any integer of 2 to 20; and i specifies the generation of a silylalkyl group represented by Xⁱ and is any integer of 1 to n when the generation number is n, wherein the generation number n is any integer of 1 to 10; ai is any integer of 0 to 2 when i is 1, and is an integer smaller than 3 when i is 2 or larger; and Xⁱ⁺¹ is the silylalkyl group when i is smaller than n, and is a methyl group when i=n; 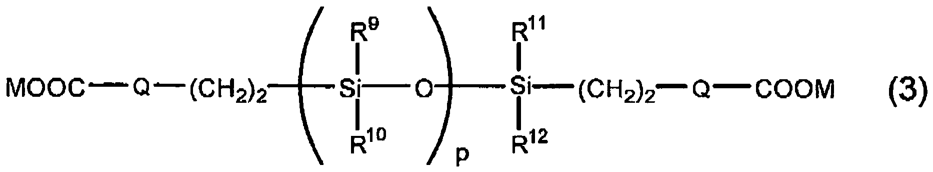 in the formula (3), R⁹ to R¹² may be the same or different and are respectively a substituted or unsubstituted monovalent hydrocarbon group; M is a divalent or trivalent metal atom; Q is a linear or branched alkylene group represented by C_{q}H_{2q} in which q is any integer of 0 to 20; and p is any number of 0 to 20.

2. A surface treating method comprising; treating a surface of a powder using a composition obtained by mixing an organosiloxane derivative having a terminal carboxyl group or a monovalent metal salt thereof, represented by the following formula (1') or (3'), with a metal salt solution containing a divalent or trivalent metal ion; 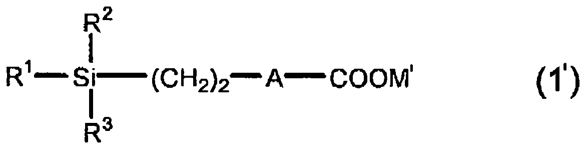 in the formula (1'), at least one of R¹ to R³ is a functional group represented by -O-Si(R⁴)₃ in which R⁴ is an alkyl group having 1 to 6 carbon atoms or a phenyl group, or a functional group represented by -O-Si(R⁵)₂-X¹ in which R⁵ is an alkyl group having 1 to 6 carbon atoms or a phenyl group, and X¹ is a functional group represented by the following formula (2) when i=1, and the remaining R¹ to R³ may be the same or different and each may be a substituted or unsubstituted monovalent hydrocarbon group; M' is a hydrogen atom or a monovalent metal atom; A is a linear or branched alkylene group represented by C_{q}H_{2q} in which q is any integer of 0 to 20; and the organosiloxane derivative represented by the formula (1') contains a total of 2 to 100 silicon atoms (Si) on average per molecule; 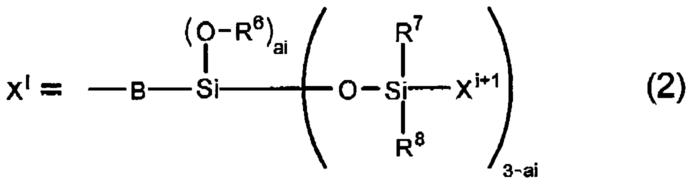 in the formula (2), R⁶ is a hydrogen atom, an alkyl group having 1 to 6 carbon atoms, or a phenyl group; R⁷ and R⁸ are respectively an alkyl group having 1 to 6 carbon atoms or a phenyl group; B is a linear or branched alkylene group represented by CᵣH₂ᵣ which may be partially branched in which r is any integer of 2 to 20; and i specifies the generation of a silylalkyl group represented by Xⁱ and is any integer of 1 to n when the generation number is n, wherein the generation number n is any integer of 1 to 10; ai is any integer of 0 to 2 when i is 1, and is an integer smaller than 3 when i is 2 or larger; and Xⁱ⁺¹ is the silylalkyl group when i is smaller than n, and is a methyl group when i=n; 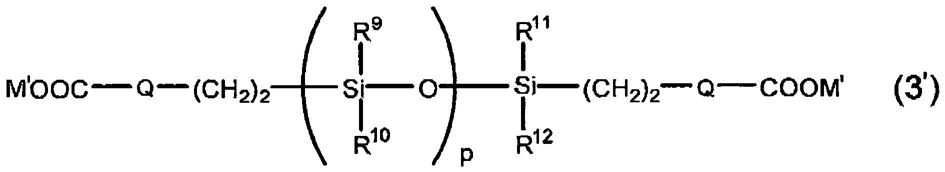 in the formula (3'), R⁹ to R¹² may be the same or different and are respectively a substituted or unsubstituted monovalent hydrocarbon group; M' is a hydrogen atom or a monovalent metal atom; Q is a linear or branched alkylene group represented by C_{q}H_{2q} in which q is any integer of 0 to 20; and p is any number of 0 to 20.

3. The surface treating method according to claim 1 or 2, wherein the organosiloxane derivative is represented by the formula (1) or (1'), R¹ and R² are respectively a functional group represented by -O-Si(R⁴)₈ in which R⁴ is an alkyl group having 1 to 6 carbon atoms; R³ is a monovalent hydrocarbon group having 1 to 10 carbon atoms; and q is any integer of 6 to 20.

4. The surface treating method according to claim 1 or 2, wherein the organosiloxane derivative is represented by the formula (1) or (1'), at least one or more of R¹ to R³ are respectively any of the functional group represented by the following formula (4) or (5), and the remaining R¹ to R³ may be the same or different and are respectively a substituted or unsubstituted monovalent hydrocarbon group.

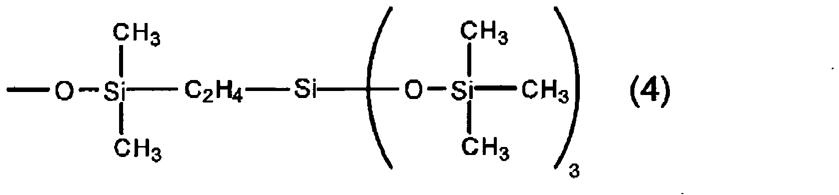

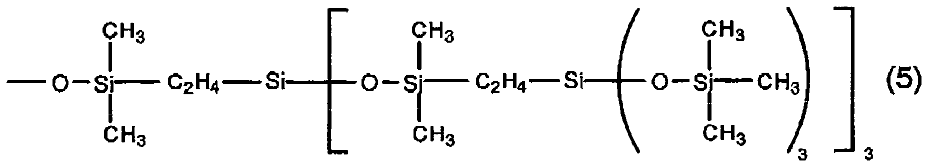

5. The surface treating method according to claim 1 or 2, wherein the organosiloxane derivative is represented by the formula (3) or (3'), R⁹ to R¹² are respectively a group selected from the group consisting of substituted or unsubstituted alkyl groups having 1 to 20 carbon atoms, aryl groups, and aralkyl groups; q is any integer of 6 to 20; and p is any number of 1 to 20.

6. A surface-treated powder being treated with a surface treating agent comprising an organosiloxane derivative having a divalent or trivalent metal salt of a terminal carboxyl group, represented by the following formula (1) or (3); 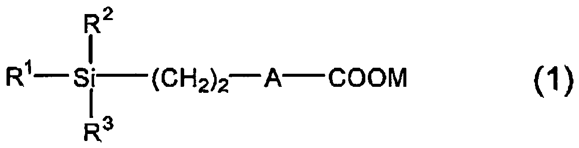 in the formula (1), at least one of R¹ to R³ is a functional group represented by -O-Si(R⁴)₃ in which R⁴ is an alkyl group having 1 to 6 carbon atoms or a phenyl group, or a functional group represented by -O-Si(R⁵)₂-X¹ in which R⁵ is an alkyl group having 1 to 6 carbon atoms or a phenyl group, and X¹ is a functional group represented by the following formula (2) when i=1; and the remaining R¹ to R³ may be the same or different and each may be a substituted or unsubstituted monovalent hydrocarbon group; M is a divalent or trivalent metal atom; A is a linear or branched alkylene group represented by C_{q}H_{2q} in which q is any integer of 0 to 20; and the organosiloxane derivative represented by the formula (1) contains a total of 2 to 100 silicon atoms (Si) on average per molecule; 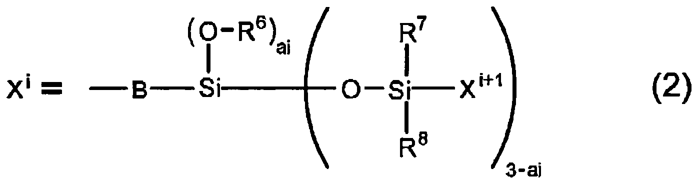 in the formula (2), R⁶ is a hydrogen atom, an alkyl group having 1 to 6 carbon atoms, or a phenyl group; R⁷ and R⁸ are respectively an alkyl group having 1 to 6 carbon atoms or a phenyl group; B is a linear or branched alkylene group represented by CᵣH₂ᵣ which may be partially branched in which r is any integer of 2 to 20; and i specifies the generation of a silylalkyl group represented by Xⁱ and is any integer of 1 to n when the generation number is n, wherein the generation number n is any integer of 1 to 10; ai is any integer of 0 to 2 when i is 1, and is an integer smaller than 3 when i is 2 or larger; and Xⁱ⁺¹ is the silylalkyl group when i is smaller than n, and is a methyl group when i=n; 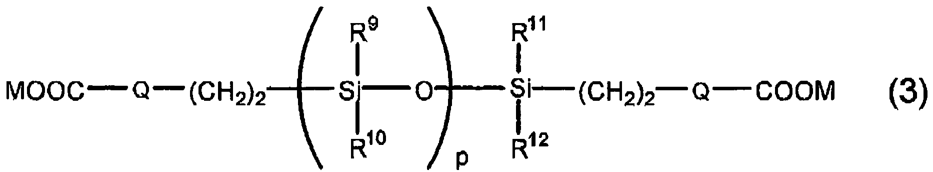 in the formula (3), R⁹ to R¹² may be the same or different and are respectively a substituted or unsubstituted monovalent hydrocarbon group; M is a divalent or trivalent metal atom; Q is a linear or branched alkylene group represented by C_{q}H_{2q} in which q is any integer of 0 to 20; and p is any number of 0 to 20.

7. A cosmetic comprising the surface-treated powder according to claim 6.

## Patentansprüche

1. Verfahren zur Oberflächenbehandlung, umfassend die Behandlung einer Oberfläche eines Pulvers mit einer Zusammensetzung, die ein Organosiloxanderivat mit einem Salz eines zwei- oder dreiwertigen Metalls einer terminalen Carboxylgruppe umfasst, wiedergegeben durch die folgende Formel (1) oder (3); 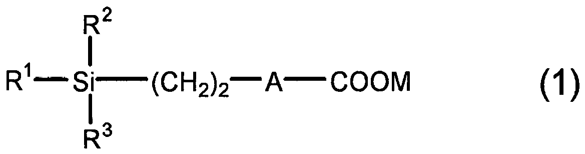 wobei in der Formel (1) mindestens einer der Reste R¹ bis R³ eine funktionelle Gruppe ist, die durch -O-Si(R⁴)₃ dargestellt ist, wobei R⁴ eine Alkylgruppe mit 1 bis 6 Kohlenstoffatomen oder eine Phenylgruppe ist, oder eine funktionelle Gruppe ist, die durch -O-Si(R⁵)₂-X¹ wiedergegeben ist, wobei R⁵ eine Alkylgruppe mit 1 bis 6 Kohlenstoffatomen oder eine Phenylgruppe und X¹ eine durch die folgende Formel (2) mit i = 1 dargestellte funktionelle Gruppe ist, und die anderen Reste R¹ bis R³ gleich oder verschieden sein können und jeweils eine substituierte oder unsubstituierte einwertige Kohlenwasserstoffgruppe sein können,
M ein zwei- oder dreiwertigen Metallatom ist,
A eine durch C_{q}H_{2q} dargestellte geradkettige oder verzweigte Alkylengruppe ist, wobei q eine beliebige ganze Zahl von 0 bis 20 ist,
und das Organosiloxanderivat nach Formel (1) insgesamt durchschnittlich 2 bis 100 Siliziumatome (Si) je Molekül enthält, 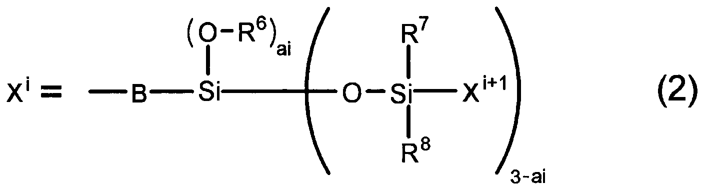 wobei in der Formel (2) R⁶ ein Wasserstoffatom, eine Alkylgruppe mit 1 bis 6 Kohlenstoffatomen oder eine Phenylgruppe ist, R⁷ und R⁸ jeweils eine Alkylgruppe mit 1 bis 6 Kohlenstoffatomen oder eine Phenylgruppe ist,
B eine durch CᵣH₂ᵣ dargestellte geradkettige oder verzweigte Alkylengruppe ist, die teilweise verzweigt sein kann, wobei r eine beliebige ganze Zahl von 2 bis 20 ist, und
i die Generation einer durch Xⁱ dargestellten Silylalkylgruppe definiert und eine beliebige ganze Zahl von 1 bis n ist, wenn n die Generationszahl ist, wobei die Generationszahl n eine beliebige ganze Zahl von 1 bis 10 ist, ai eine beliebige ganze Zahl von 0 bis 2 ist, wenn i 1 ist, und eine ganze Zahl kleiner als 3 ist, wenn i 2 oder größer ist,
und Xⁱ⁺¹ die Silylalkylgruppe ist, wenn i kleiner als n ist und eine Methylgruppe ist, wenn i = n ist, 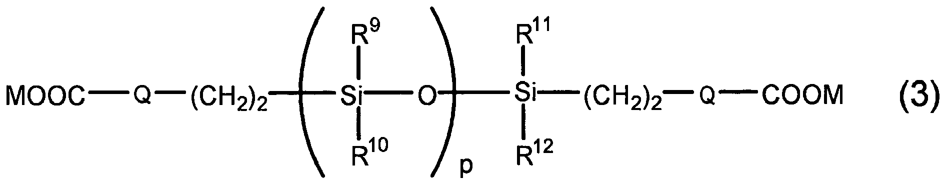 wobei in der Formel (3) R⁹ bis R¹² gleich oder verschieden sein können und jeweils eine substituierte oder unsubstituierte einwertige Kohlenwasserstoffgruppe sind,
M ein zweiwertiges oder dreiwertiges Metallatom ist,
Q eine durch C_{q}H_{2q} dargestellte geradkettige oder verzweigte Alkylengruppe ist, wobei q eine beliebige ganze Zahl von 0 bis 20 ist, und P eine beliebige Zahl von 0 bis 20 ist.

2. Verfahren zur Oberflächenbehandlung, umfassend die Behandlung einer Oberfläche eines Pulvers unter Verwendung einer Zusammensetzung, die durch Vermischen eines Organosiloxanderivats mit einer terminalen Carboxylgruppe oder einem Salz eines einwertigen Metalls davon, dargestellt durch die folgende Formel (1') oder (3'), mit einer Metallsalzlösung, die ein zweiwertiges oder dreiwertiges Metallsalz enthält, erhalten wird, 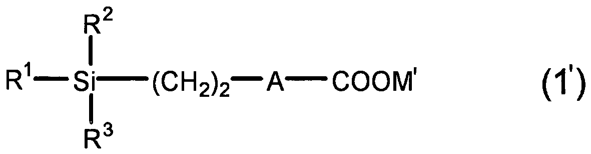 wobei in der Formel (1') mindestens einer der Reste R¹ bis R³ eine funktionelle Gruppe ist, die durch -O-Si(R⁴)₃ dargestellt ist, wobei R⁴ eine Alkylgruppe mit 1 bis 6 Kohlenstoffatomen oder eine Phenylgruppe ist, oder eine funktionelle Gruppe ist, die durch -O-Si(R⁵)₂-X¹ wiedergegeben ist, wobei R⁵ eine Alkylgruppe mit 1 bis 6 Kohlenstoffatomen oder eine Phenylgruppe und X¹ eine durch die folgende Formel (2) mit i = 1 dargestellte funktionelle Gruppe ist, und die anderen Reste R¹ bis R³ gleich oder verschieden sein können und jeweils eine substituierte oder unsubstituierte einwertige Kohlenwasserstoffgruppe sein können,
M' ein Wasserstoffatom oder ein einwertige Metallatom ist,
A eine durch C_{q}H_{2q} dargestellte geradkettige oder verzweigte Alkylengruppe ist, wobei q eine beliebige ganze Zahl von 0 bis 20 ist,
und das Organosiloxanderivat nach Formel (1') insgesamt durchschnittlich 2 bis 100 Siliziumsatome (Si) je Molekül enthält, 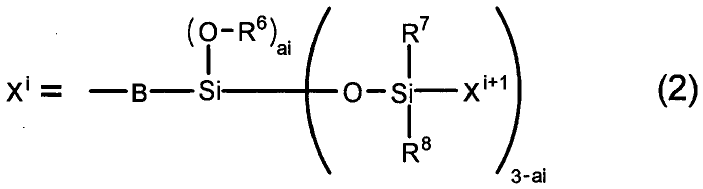 wobei in der Formel (2) R⁶ ein Wasserstoffatom, eine Alkylgruppe mit 1 bis 6 Kohlenstoffatomen oder eine Phenylgruppe ist, R⁷ und R⁸ jeweils eine Alkylgruppe mit 1 bis 6 Kohlenstoffatomen oder eine Phenylgruppe ist,
B eine durch CᵣH₂ᵣ dargestellte geradkettige oder verzweigte Alkylengruppe ist, die teilweise verzweigt sein kann, wobei r eine beliebige ganze Zahl von 2 bis 20 ist, und
i die Generation einer durch Xⁱ dargestellten Silylalkylgruppe definiert und eine beliebige ganze Zahl von 1 bis n ist, wenn n die Generationszahl ist, wobei die Generationszahl n eine beliebige ganze Zahl von 1 bis 10 ist, ai eine beliebige ganze Zahl von 0 bis 2 ist, wenn i 1 ist, und eine ganze Zahl kleiner als 3 ist, wenn i 2 oder größer ist,
und Xⁱ⁺¹ die Silylalkylgruppe ist, wenn i kleiner als n ist und eine Methylgruppe, wenn i = n ist, 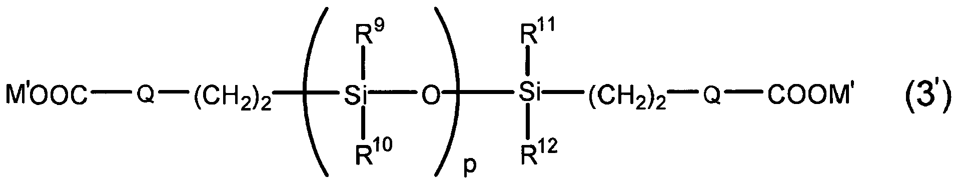 wobei in der Formel (3')R⁹ bis R¹² gleich oder verschieden sein können und jeweils eine substituierte oder unsubstituierte einwertige Kohlenwasserstoffgruppe sind,
M' ein Wasserstoffatom oder ein einwertiges Metallatom ist,
Q eine durch C_{q}H_{2q} dargestellte geradkettige oder verzweigte Alkylengruppe ist, wobei q eine beliebige ganze Zahl von 0 bis 20 ist, und P eine beliebige Zahl von von 0 bis 20 ist.

3. Verfahren zur Oberflächenbehandlung nach Anspruch 1 oder 2, wobei das Organosiloxanderivat durch die Formel (1) oder (1') wiedergegeben wird, R¹ bzw. R² eine durch -O-Si(R⁴)₃ dargestellte funktionelle Gruppe ist, wobei R⁴ eine Alkylgruppe mit 1 bis 6 Kohlenstoffatomen ist, R³ eine einwertige Kohlenwasserstoffgruppe mit 1 bis 10 Kohlenstoffatomen ist, und q eine beliebige ganze Zahl von 6 bis 20 ist.

4. Verfahren zur Oberflächenbehandlung nach Anspruch 1 oder 2, wobei das Organosiloxanderivat durch Formel (1) oder (1') wiedergegeben wird, mindestens einer der Reste R¹ bis R³ eine beliebige der durch die folgenden Formeln (4) oder (5) wiedergegebenen funktionellen Gruppen ist und die anderen R¹ bis R³ gleich oder verschieden sein können und jeweils eine substituierte oder unsubstituierten einwertige Kohlenwasserstoffgruppe sind.

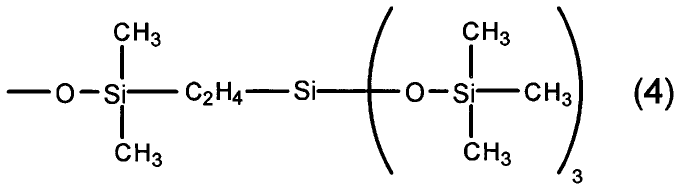

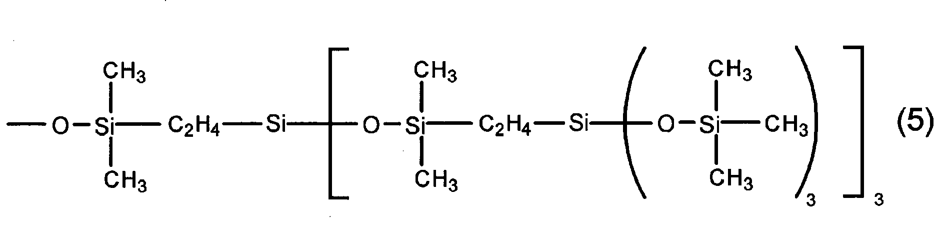

5. Verfahren zur Oberflächenbehandlung nach Anspruch 1 oder 2, wobei das Organosiloxanderivat durch die Formeln (3) oder (3') dargestellt wird, R⁹ bis R¹² jeweils eine aus der aus substituierten oder unsubstituierten Alkylgruppen mit 1 bis 20 Kohlenstoffatomen, Arylgruppen und Aralkylgruppen bestehenden Gruppe ausgewählte Gruppe ist, q eine beliebige ganze Zahl von 6 bis 20 ist und p eine beliebige Zahl von 1 bis 20 ist.

6. Oberflächenbehandeltes Pulver, das mit einem Mittel zur Oberflächenbehandlung behandelt wurde, das ein Organosiloxanderivat mit einem Salz eines zwei- oder dreiwertigen Metalls einer terminalen Carboxylgruppe umfasst, wiedergegeben durch die folgende Formel (1) oder (3), 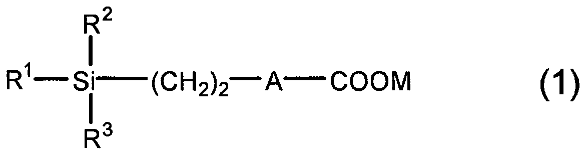 wobei in der Formel (1) mindestens einer der Reste R¹ bis R³ eine funktionelle Gruppe ist, die durch -O-Si(R⁴)₃ dargestellt ist, wobei R⁴ eine Alkylgruppe mit 1 bis 6 Kohlenstoffatomen oder eine Phenylgruppe ist, oder eine funktionelle Gruppe ist, die durch -O-Si(R⁵)₂-X¹ wiedergegeben ist, wobei R⁵ eine Alkylgruppe mit 1 bis 6 Kohlenstoffatomen oder eine Phenylgruppe ist und X¹ eine durch die folgende Formel (2) mit i = 1 dargestellte funktionelle Gruppe ist, und die anderen Reste R¹ bis R³ gleich oder verschieden sein können und jeweils eine substituierte oder unsubstituierte einwertige Kohlenwasserstoffgruppe sein können,
M ein zwei- oder dreiwertigen Metallatom ist,
A eine durch C_{q}H_{2q} dargestellte geradkettige oder verzweigte Alkylengruppe ist, wobei q eine beliebige ganze Zahl von 0 bis 20 ist,
und das Organosiloxanderivat nach Formel (1) insgesamt durchschnittlich 2 bis 100 Siliziumatome (Si) je Molekül enthält, 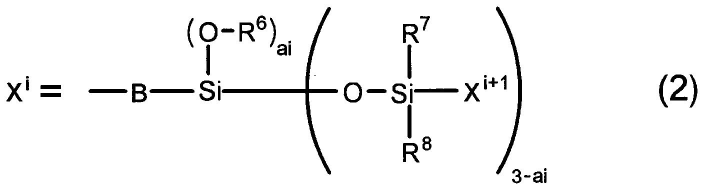 wobei in der Formel (2) R⁶ ein Wasserstoffatom, eine Alkylgruppe mit 1 bis 6 Kohlenstoffatomen oder eine Phenylgruppe ist, R⁷ und R⁸ jeweils eine Alkylgruppe mit 1 bis 6 Kohlenstoffatomen oder eine Phenylgruppe ist,
B eine durch CᵣH₂ᵣ dargestellte geradkettige oder verzweigte Alkylengruppe ist, die teilweise verzweigt sein kann, wobei r eine beliebige ganze Zahl von 2 bis 20 ist, und
i die Generation einer durch Xⁱ dargestellten Silylalkylgruppe definiert und eine beliebige ganze Zahl von 1 bis n ist, wenn n die Generationszahl ist, wobei die Generationszahl n eine beliebige ganze Zahl von 1 bis 10 ist, ai eine beliebige ganze Zahl von 0 bis 2 ist, wenn i 1 ist, und eine ganze Zahl kleiner als 3 ist, wenn i 2 oder größer ist,
und Xⁱ⁺¹ die Silylalkylgruppe ist, wenn i kleiner als n ist, und eine Methylgruppe ist, wenn i = n ist, 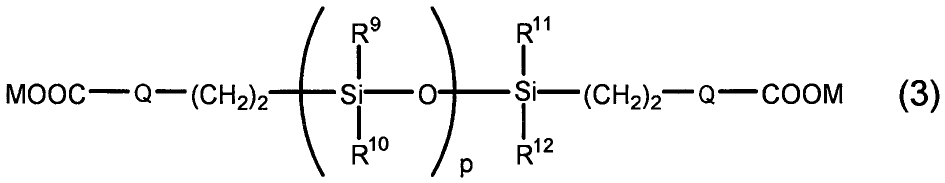 wobei in der Formel (3) R⁹ bis R¹² gleich oder verschieden sein können und jeweils eine substituierte oder unsubstituierte einwertige Kohlenwasserstoffgruppe sind,
M ein zweiwertiges oder dreiwertiges Metallatom ist,
Q eine durch C_{q}H_{2q} dargestellte geradkettige oder verzweigte Alkylengruppe ist, wobei q eine beliebige ganze Zahl von 0 bis 20 ist, und P eine beliebige Zahl von 0 bis 20 ist.

7. Kosmetische Zubereitung, enthaltend das oberflächenbehandelte Pulver nach Anspruch 6.

## Revendications

1. Procédé de traitement de surface comprenant :
le traitement d'une surface d'une poudre à l'aide d'une composition comprenant un dérivé d'organosiloxane contenant un sel métallique divalent ou trivalent d'un groupe carboxyle terminal, représenté par la formule (1) ou (3) suivante : 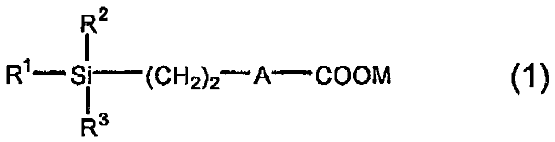 dans la formule (1), l'un au moins de R¹ à R³ est un groupe fonctionnel représenté par -O-Si(R⁴)₃ où R⁴ est un groupe alkyle comportant 1 à 6 atomes de carbone ou un groupe phényle, ou un groupe fonctionnel représenté par -O-Si(R⁵)₂-X¹ où R⁵ est un groupe alkyle comportant 1 à 6 atomes de carbone ou un groupe phényle, et X¹ est un groupe fonctionnel représenté par la formule (2) suivante lorsque i=1 ; et les groupes R¹ à R³ restants peuvent être identiques ou différents et peuvent être chacun un groupe hydrocarboné monovalent substitué ou non substitué ; M est un atome de métal divalent ou trivalent ; A est un groupe alkylène linéaire ou ramifié représenté par C_{q}H_{2q} où q est tout nombre entier de 0 à 20 ; et le dérivé d'organosiloxane représenté par la formule (1) contient un total de 2 à 100 atomes de silicium (Si) en moyenne par molécule ; 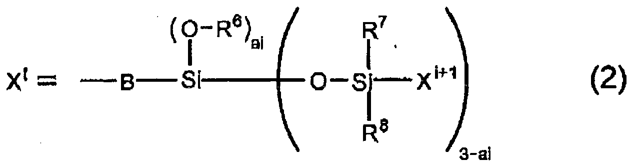 dans la formule (2), R⁶ est un atome d'hydrogène, un groupe alkyle comportant 1 à 6 atomes de carbone ou un groupe phényle ; R⁷ et R⁸ sont respectivement un groupe alkyle comportant 1 à 6 atomes de carbone ou un groupe phényle ; B est un groupe alkylène linéaire ou ramifié représenté par CᵣH₂ᵣ qui peut être partiellement ramifié où r est tout nombre entier de 2 à 20 ; et i désigne la génération d'un groupe silylalkyle représenté par Xⁱ et est tout nombre entier de 1 à n lorsque le nombre de génération est n, où le nombre de génération n est tout nombre entier de 1 à 10 ; ai est tout nombre entier de 0 à 2 lorsque i vaut 1 et est un nombre entier inférieur à 3 lorsque i est supérieur ou égal à 2 ; et Xⁱ⁺¹ est le groupe silylalkyle lorsque i est inférieur à n et est un groupe méthyle lorsque i=n ; 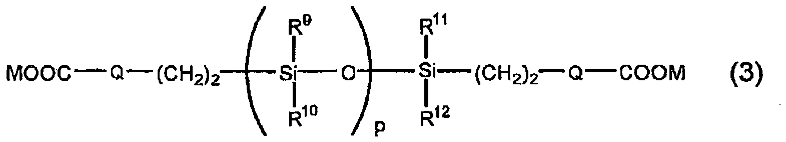 dans la formule (3), R⁹ à R¹² peuvent être identiques ou différents et sont respectivement un groupe hydrocarboné monovalent substitué ou non substitué ; M est un atome de métal divalent ou trivalent ; Q est un groupe alkylène linéaire ou ramifié représenté par C_{q}H_{2q} où q est tout nombre entier de 0 à 20 ; et p est tout nombre entier de 0 à 20.

2. Procédé de traitement de surface comprenant :
le traitement d'une surface d'une poudre à l'aide d'une composition obtenue par mélange d'un dérivé d'organosiloxane contenant un groupe carboxyle terminal ou un sel métallique monovalent de celui-ci, représenté par la formule (1') ou (3') suivante, avec une solution d'un sel métallique contenant un ion métallique divalent ou trivalent : 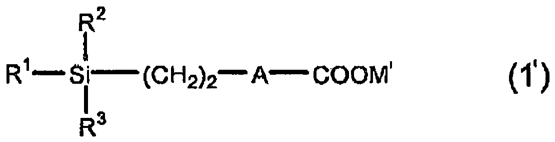 dans la formule (1'), l'un au moins de R¹ à R³ est un groupe fonctionnel représenté par -O-Si(R⁴)₃ où R⁴ est un groupe alkyle comportant 1 à 6 atomes de carbone ou un groupe phényle, ou un groupe fonctionnel représenté par -O-Si(R⁵)₂-X¹ où R⁵ est un groupe alkyle comportant 1 à 6 atomes de carbone ou un groupe phényle, et X¹ est un groupe fonctionnel représenté par la formule (2) suivante lorsque i=1, et les groupes R¹ à R³ restants peuvent être identiques ou différents et peuvent être chacun un groupe hydrocarboné monovalent substitué ou non substitué ; M' est un atome d'hydrogène ou un atome de métal monovalent ; A est un groupe alkylène linéaire ou ramifié représenté par C_{q}H_{2q} où q est tout nombre entier de 0 à 20 ; et le dérivé d'organosiloxane représenté par la formule (1') contient un total de 2 à 100 atomes de silicium (Si) en moyenne par molécule ; 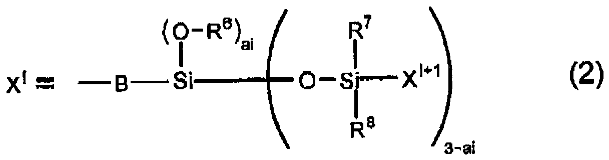 dans la formule (2), R⁶ est un atome d'hydrogène, un groupe alkyle comportant 1 à 6 atomes de carbone ou un groupe phényle ; R⁷ et R⁸ sont respectivement un groupe alkyle comportant 1 à 6 atomes de carbone ou un groupe phényle ; B est un groupe alkylène linéaire ou ramifié représenté par CᵣH₂ᵣ qui peut être partiellement ramifié où r est tout nombre entier de 2 à 20 ; et i désigne la génération d'un groupe silylalkyle représenté par Xⁱ et est tout nombre entier de 1 à n lorsque le nombre de génération est n, où le nombre de génération n est tout nombre entier de 1 à 10 ; ai est tout nombre entier de 0 à 2 lorsque i vaut 1 et est un nombre entier inférieur à 3 lorsque i est supérieur ou égal à 2 ; et Xⁱ⁺¹ est le groupe silylalkyle lorsque i est inférieur à n et est un groupe méthyle lorsque i=n ; 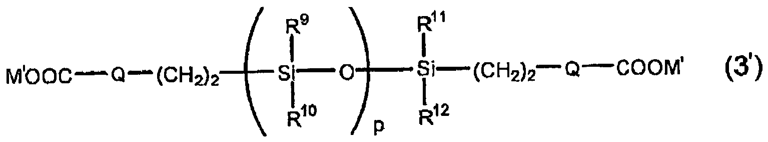 dans la formule (3'), R⁹ à R¹² peuvent être identiques ou différents et sont respectivement un groupe hydrocarboné monovalent substitué ou non substitué ; M' est un atome d'hydrogène ou un atome de métal monovalent ; Q est un groupe alkylène linéaire ou ramifié représenté par C_{q}H_{2q} où q est tout nombre entier de 0 à 20 ; et p est tout nombre entier de 0 à 20.

3. Procédé de traitement de surface selon la revendication 1 ou 2, dans lequel le dérivé d'organosiloxane est représenté par la formule (1) ou (1'), R¹ et R² sont respectivement un groupe fonctionnel représenté par -O-Si(R⁴)₃ où R⁴ est un groupe alkyle comportant 1 à 6 atomes de carbone ; R³ est un groupe hydrocarboné monovalent comportant 1 à 10 atomes de carbone ; et q est tout nombre entier de 6 à 20.

4. Procédé de traitement de surface selon la revendication 1 ou 2, dans lequel le dérivé d'organosiloxane est représenté par la formule (1) ou (1'), l'un au moins ou plusieurs parmi R¹ à R³ sont respectivement un quelconque groupe fonctionnel représenté par la formule (4) ou (5) suivante et les groupes R¹ à R³ restants peuvent être identiques ou différents et sont respectivement un groupe hydrocarboné monovalent substitué ou non substitué.

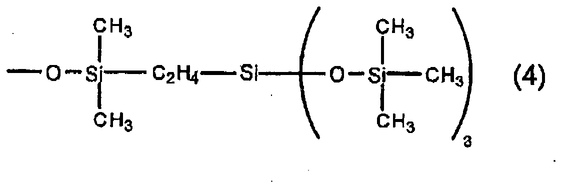

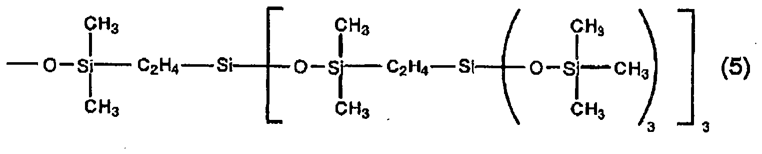

5. Procédé de traitement de surface selon la revendication 1 ou 2, dans lequel le dérivé d'organosiloxane est représenté par la formule (3) ou (3'), R⁹ à R¹² sont respectivement un groupe choisi dans le groupe constitué des groupes alkyle substitués ou non substitués comportant 1 à 20 atomes de carbone, des groupes aryle et des groupes aralkyle ; q est tout nombre entier de 6 à 20 ; et p est tout nombre entier de 1 à 20.

6. Poudre traitée en surface, traitée avec un agent de traitement de surface comprenant un dérivé d'organosiloxane contenant un sel métallique divalent ou trivalent d'un groupe carboxyle terminal, représenté par la formule (1) ou (3) suivante : 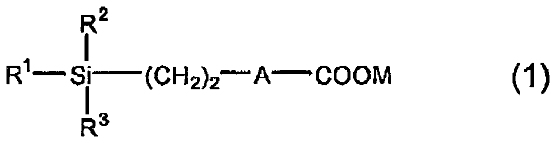 dans la formule (1), l'un au moins de R¹ à R³ est un groupe fonctionnel représenté par -O-Si(R⁴)₃ où R⁴ est un groupe alkyle comportant 1 à 6 atomes de carbone ou un groupe phényle, ou un groupe fonctionnel représenté par -O-Si(R⁵)₂-X¹ où R⁵ est un groupe alkyle comportant 1 à 6 atomes de carbone ou un groupe phényle, et X¹ est un groupe fonctionnel représenté par la formule (2) suivante lorsque i=1 ; et les groupes R¹ à R³ restants peuvent être identiques ou différents et peuvent être chacun un groupe hydrocarboné monovalent substitué ou non substitué ; M est un atome de métal divalent ou trivalent ; A est un groupe alkylène linéaire ou ramifié représenté par C_{q}H_{2q} où q est tout nombre entier de 0 à 20 ; et le dérivé d'organosiloxane représenté par la formule (1) contient un total de 2 à 100 atomes de silicium (Si) en moyenne par molécule ; 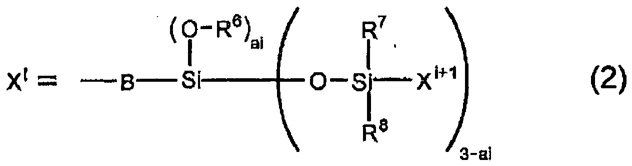 dans la formule (2), R⁶ est un atome d'hydrogène, un groupe alkyle comportant 1 à 6 atomes de carbone ou un groupe phényle ; R⁷ et R⁸ sont respectivement un groupe alkyle comportant 1 à 6 atomes de carbone ou un groupe phényle ; B est un groupe alkylène linéaire ou ramifié représenté par CᵣH₂ᵣ qui peut être partiellement ramifié où r est tout nombre entier de 2 à 20 ; et i désigne la génération d'un groupe silylalkyle représenté par Xⁱ et est tout nombre entier de 1 à n lorsque le nombre de génération est n, où le nombre de génération n est tout nombre entier de 1 à 10 ; ai est tout nombre entier de 0 à 2 lorsque i vaut 1 et est un nombre entier inférieur à 3 lorsque i est supérieur ou égal à 2 ; et Xⁱ⁺¹ est le groupe silylalkyle lorsque i est inférieur à n et est un groupe méthyle lorsque i=n ; 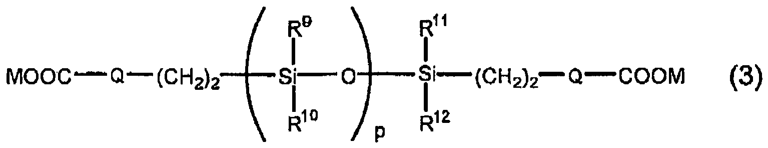 dans la formule (3), R⁹ à R¹² peuvent être identiques ou différents et sont respectivement un groupe hydrocarboné monovalent substitué ou non substitué ; M est un atome de métal divalent ou trivalent ; Q est un groupe alkylène linéaire ou ramifié représenté par C_{q}H_{2q} où q est tout nombre entier de 0 à 20 ; et p est tout nombre entier de 0 à 20.

7. Produit cosmétique comprenant la poudre traitée en surface selon la revendication 6.
